# EUROPEAN PATENT SPECIFICATION

(11) **EP 1 627 074 B1**
(45) Date of publication and mention of the grant of the patent: **09.07.2008**
(21) Application number: 03723302.0
(22) Date of filing: 09.05.2003
(51) Int. Cl.: C12Q 1/68, C12N 15/10, C07H 21/00

(54) **USE OF A TYPE III RESTRICTION ENZYME TO ISOLATE SEQUENCE TAGS COMPRISING MORE THAN 25 NUCLEOTIDES**
VERWENDUNG EINES TYP-III-RESTRIKTIONSENZYMS ZUR ISOLIERUNG VON MEHR ALS 25 NUKLEOTIDE UMFASSENDEN SEQUENZ-TAGS
UTILISATION D'UNE ENZYME DE RESTRICTION DE TYPE III POUR ISOLER DES ETIQUETTES DE SEQUENCE COMPRENANT PLUS DE 25 NUCLEOTIDES

(43) Date of publication of application: 22.02.2006
(73) Proprietor: Winter, Peter, 65779 Kelkheim/Ts. (DE)
(72) Inventor: WINTER, Peter, 65779 Kelkheim/Ts. (DE); KAHL, Guenter, 63500 Seligenstadt (DE); KRUEGER, Detlev, 14532 Stahnsdorf (DE); REICH, Stefanie, Dr., Dept of Crystallography,, Malet Street, London WC1E 7HX (GB); MATSUMURA, Hideo, Kitakami-shi, Iwate 024-0061 (JP); TERAUCHI, Ryohei, Narita 22-174-4,Kitakami-Iwate 024-0003 (JP)
(74) Representative: Neuefeind, Regina
(86) International application number: PCT/JP2003/005840
(87) International publication number: WO 2004/099445

(56) References cited:
- TANAKA Y ET AL: "New genotypes of TT virus (TTV) and a genotyping assay based on restriction fragment length polymorphism" FEBS LETTERS, ELSEVIER SCIENCE PUBLISHERS, AMSTERDAM, NL, vol. 437, no. 3, 23 October 1998 (1998-10-23), pages 201-206, XP004258524 ISSN: 0014-5793
- VELCULESCU V E ET AL: "SERIAL ANALYSIS OF GENE EXPRESSION" SCIENCE, AMERICAN ASSOCIATION FOR THE ADVANCEMENT OF SCIENCE,, US, vol. 270, no. 5235, 20 October 1995 (1995-10-20), pages 484-487, XP001024449 ISSN: 0036-8075 cited in the application
- YAMAMOTO M ET AL: "Use of serial analysis of gene expression (SAGE) technology" JOURNAL OF IMMUNOLOGICAL METHODS, ELSEVIER SCIENCE PUBLISHERS B.V.,AMSTERDAM, NL, vol. 250, no. 1-2, 1 April 2001 (2001-04-01), pages 45-66, XP004230694 ISSN: 0022-1759
- PLEASANCE ERIN D ET AL: "Assessment of SAGE in transcript identification." GENOME RESEARCH, vol. 13, no. 6a, June 2003 (2003-06), pages 1203-1215, XP002267288 ISSN: 1088-9051 (ISSN print)
- SAHA SAURABH ET AL: "Using the transcriptome to annotate the genome" NATURE BIOTECHNOLOGY, vol. 19, no. 5, May 2002 (2002-05), pages 508-512, XP001157218 ISSN: 1087-0156 cited in the application

### SEQUENCE LISTING

<110> Iwate Prefectural Government
   KAHL, Guenter
   WINTER, Peter
   KRUEGER, Detlev
   REICH, Stefanie
<120> USE 0F A TYPE III RESTRICTION ENZYME T0 ISOLATE IDENTIFICATION TAGS COMPRISING MORE THAN 25 NUCLEOTIDES
<130> PH-1719-PCT
<140>
   <141>
<160> 9
<170> PatentIn Ver. 2.1
<210> 1
   <211> 47
   <212> DNA
   <213> Artificial Sequence
<220>
   <223> Inventor: KAHL, Guenter; WINTER, Peter; KRUEGER, Detlev; REICH, Stefanie; MATSUMURA, Hideo; TERAUCHI, Ryohei
<220>
   <223> Description of Artificial Sequence:RT-primer
<400>1
   ctgatctaga ggtaccggat cccagcagtt tttttttttt ttttttt 47
<210> 2
   <211> 46
   <212> DNA
   <213> Artificial Sequence
<220>
   <223> Description of Artificial Sequence:first strand of Linker-A
<400> 2
   tttggatttg ctggtgcagt acaactaggc ttaatacagc agcatg 46
<210> 3
   <211> 42
   <212> DNA
   <213> Artificial Sequence
<220>
   <223> Description of Artificial Sequence:second strand of Linker-A
<400> 3
   ctgctgtatt aagcctagtt gtactgcacc agcaaatcca aa 42
<210> 4
   <211> 44
   <212> DNA
   <213> Artificial Sequence
<220>
   <223> Description of Artificial Sequence:first strand of Linker-B
<400> 4
   tttctgctcg aattcaagct tctaacgatg tacgcagcag catg 44
<210> 5
   <211> 40
   <212> DNA
   <213> Artificial Sequence
<220>
   <223> Description of Artificial Sequence:second strand of Linker-B
<400> 5
   ctgctgcgta catcgttaga agcttgaatt cgagcagaaa 40
<210> 6
   <211> 23
   <212> DNA
   <213> Artificial Sequence
<220>
   <223> Description of Artificial Sequence:Ditag primer (forward)
<400> 6
   caactaggct taatacagca gca 23
<210> 7
   <211> 21
   <212> DNA
   <213> Artificial Sequence
<220>
   <223> Description of Artificial Sequence:Ditag primer (reverse)
<400> 7
   ctaacgatgt acgcagcagc a 21
<210> 8
   <211> 37
   <212> DNA
   <213> Artificial Sequence
<220>
   <223> Description of Artificial Sequence:RT-primer
<400> 8
   ggccacgcgt cgactagtac tttttttttt ttttttt 37
<210> 9
   <211> 20
   <212> DNA
   <213> Artificial Sequence
<220>
   <223> Description of Artificial Sequence:primer for 3'-RACE
<400> 9
   ggccacgcgt cgactagtac 20

## Description

### TECHNICAL FIELD

The present invention relates to the field of molecular biology and analysis of gene expression. In this context, it concerns the use of a type III restriction enzyme for isolating a defined region of a transcript

### BACKGROUND ART

Modification of the structure and expression of genes by genetic engineering provides enormous potential for various medical, pharmaceutical and agricultural applications. In order to detect and select target genes for engineering, screening of cells, tissues, organs or organisms in different developmental stages and under a variety of environmental conditions (such as stress) by expression profiling is extensively applied. One of the most powerful techniques for gene expression analysis is SAGE^{™} (Serial Analysis of Gene Expression) as developed by Velculescu et al., Science 270: 484-487, 1995.

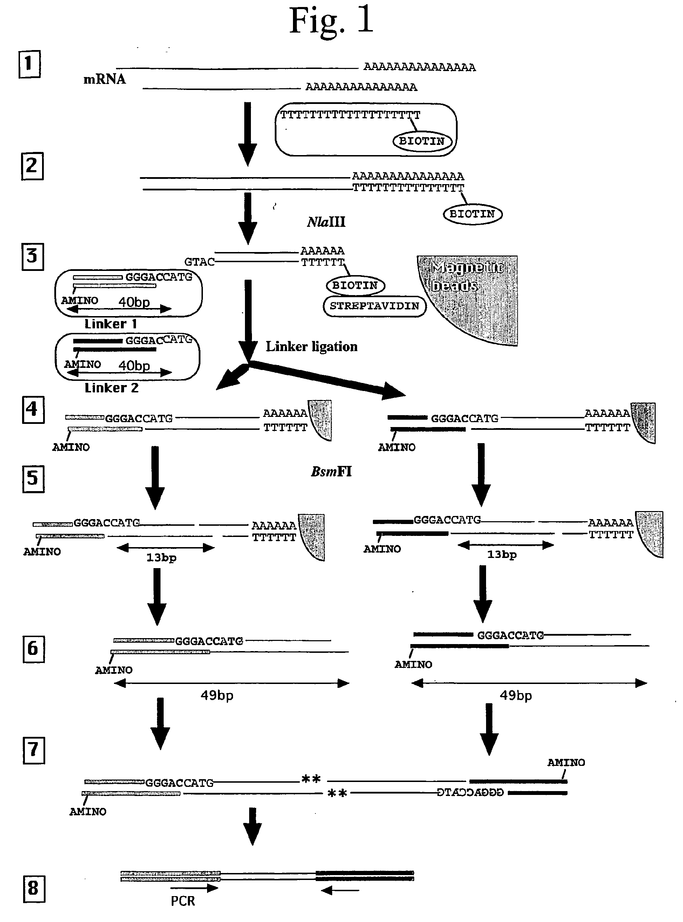

In the original SAGE^{™} protocol (Fig. 1), a pool of messenger RNA (mRNA) from defined cells, tissues or organs is used as the source material, from which complementary DNA (cDNA) is reversely transcribed by reverse transcriptase using a biotinylated oligo-dT primer. The generated single-stranded cDNA is converted into double-stranded DNA, and digested with restriction enzyme *Nla*III, which recognizes the sequence motif 5'-CATG-3'. Streptavidin-coated magnetic beads are used to recover the 3' -end fragments of the double-stranded cDNA. The cDNA is divided into two portions. Two linkers (Linker 1 and Linker 2) are then ligated to each of the cDNA portions. The linkers contain the sequence motif 5'-GGGAC-3'. This is the recognition site of the type II restriction enzyme *Bsm*FI, which cleaves 13 bp apart from the recognition site in the 3'- direction. Thus, treatment of the linkered cDNAs with *Bsm*F1 releases a 13-bp fragment of the cDNA, called the "tag" sequence, together with the linker fragment ("linker-tag" fragment). Then two of the linker-tag fragments generated from two portions of cDNAs are ligated to each other in such a manner that the two tags are in adjacent position to form a ditag, followed by PCR with primers specific to the linker sequence. After removing the linker fragment by *Nla*III digestion, the ditags are concatenated, and cloned into an appropriate plasmid. Sequencing of the plasmid insert shows a series of 9bp tags flanked by the 4-bp 5'-CATG-3' sequence. Using the 13-bp tag sequence, in many cases it is possible to identify the gene from which a tag sequence originated, by consulting available expressed sequence tag (EST) databases. Thus, after sequencing thousands of tags, it is possible to count the number of each tag in the sample, and further identify the genes corresponding thereto.

The SAGE^{™} protocol described above is therefore an effective method to study global gene expression. However, the limited size of the tag sequence (only 13 bp) is not sufficient to unequivocally identify the gene from which the tag was derived. A single tag sequence may correspond to several different EST sequences, and may confound further analysis.

To improve the situation, a so-called "LongSAGE^{™}" protocol was recently developed, involving the type II restriction endonuclease *Mme*I. With the use of *Mme*I (instead of *Bsm*FI as in the original SAGE^{™} protocol), it is possible to recover 19 to 21-bp-long tags (Saha et al. Nature Biotechnology 20: 508-512, 2002).

*Mme*I digestion produces 3'-protruding ends (2-base protrusions). To make blunt ends after *Mme*I digestion before ditag ligation, one must remove the 3'-protrusion. Currently there is no single enzyme available for filling in the 3'-protrusion. Removal of the 3'-protrusion necessary for blunting results in the reduction of the length of tag sequence to 17-19 bp, causing reduction in the information contained in the tag sequence.

Therefore, in the published LongSAGE^{™} protocol (Saha et al., Nature Biotechnology 20: 508-512, 2002), the ends after *Mme*I digestion are not polished, and ligation is made between the fragments with 3'-protrusions. This means that a linker-tag fragment ligates itself only with another linker-tag fragment harboring the compatible 3'-end. This entails that ditags formed after ligation are not the results of random association of tags. This procedure theoretically skews the representation of each tag in resulting ditags, and the final result of LongSAGE^{™} may not faithfully reflect the abundance of expression of each gene. Therefore, LongSAGE^{™} is not applicable for accurate quantitative analysis of gene expression. This represents a serious drawback of LongSAGE^{™}. For this reason, the current LongSAGE^{™} is only used to help annotate the 13-bp tag sequences obtained by the conventional SAGE^{™} protocol.

The LongSAGE^{™} protocol is a step forward, because it increases the possibility of successful identification of the genes corresponding to the tags, given that EST and/or genomic DNA sequence information is available. However, for an application of the SAGE^{™} protocol to organisms, for which no EST or genomic DNA database is available, it is imperative to use the tag sequence as a primer to recover the longer cDNA by PCR, or as an oligonucleotide probe to screen a relevant cDNA library by hybridization-based techniques. For these purposes, tag lengths of 19-21 bp are still too short to unequivocally identify the gene from which a tag sequence originated.

Therefore, there is an acute need for a method to isolate noticeably longer tags of at least 25 bp in length, with which accurate quantitative analysis of expression can be performed. However, such a method has not been available to date.

### SUMMARY OF THE INVENTION

The present invention provides a method for isolating "tag" sequences of more than 25 bp long, preferably 26 to 50 bp long, and most preferably 26 to 28 bp long, from defined positions of DNAs, thereby increasing the efficiency to reliably identify the corresponding genes by conventional SAGE^{™} analysis. Moreover, the gene expression profiles obtained by this method are theoretically more accurate than those obtained from LongSAGE analysis, since the ditags are made by the random association of tags. We hereinafter term this improved SAGE^{™} procedure with new tag fragments of more than 25 bp as "SuperSAGE".

The method described herein is based on the use of a type III restriction enzyme , for the isolation of tag sequences of more than 25 bp in length from a defined position of a cDNA. In the present invention, the term tag refers to a specific nucleotide sequence capable of identifying a expressed gene. The 3' end of the tag is defined by the cleavage site of the type III restriction enzyme, and the 5' end of the tag is defined by the cleavage site of another restriction enzyme that is closest to the 3' end of the cDNA.

"Restriction enzyme" is a general term for endonuclease capable of recognizing a specific sequence of 4 to 8 nucleotides in double-stranded DNA and cleaving it Restriction enzymes are currently classified into four different groups, called type I, II, III, and IV (Roberts et al. 2003, Nucleic Acids Res. 31: 1805-1812). Type III restriction enzymes are complex proteins consisting of methylase and endonuclease subunits and recognizing non palindromic nucleotide sequences in the target DNA. For endonucleolytic activity, they require the functional cooperation with two copies of the recognition sequence and, moreover, these two copies need to be inversely oriented within the DNA double strand (Muecke et al. 2001, J. Molec. Biol. 312: 687-698).

In the present invention, the above type III restriction enzymes are used for the isolation of tag sequences more than 25 bp in length. Examples of such type III enzymes are disclosed in http://rebase.neb.com/cgi-bin/azlist?re3. The preferred type III enzymes used in the invention include *Eco*PI, *Eco*P15I, and the like.

In the most preferred embodiment of the invention, the type III restriction enzyme is *Eco*P15I. The type III restriction enzyme *Eco*P15I recognizes two unmethylated inversely oriented 5'-**CAGCAG**-3' sites in the target DNA molecule, and digests 25 to 28 bp apart from the 3'-end of one of the recognition sites. Thus, *Eco*P15I provides fragments having an overhanging 5' end which is easily blunt-ended using a conventional 3' filling reaction.

The preferred example of the other enzyme is an enzyme capable of cleaving cDNA into fragments with an average length of 200 bp to 300 bp each, such as:

| recognition seq | Enzyme Name (commercially available only) |
|---|---|
| CATG^ | NlaIII, Hsp92II, |
| ^CATG | FatI |
| C^TAG | Bfa I, MaeI, XspI |
| A^CGT | HpyCH4IV, MaeII, |
| ACGT^ | TaiI, TscI |
| AG^CT | AluI |
| T^CGA | TaqI |
| ^GATC | BfuCI, Bsp143I, BstENII, DpnII, Kzo9I, MboI, NdeII, Sau3AI |
| GAT^C | BstKTI, |
| G^TAC | Csp6I |

The most preferable enzyme is *Nla*III.

Also disclosed is a tag comprising more than 25 nucleotides and capable of identifying an expressed gene, wherein the 3' end of the tag is defined by a cleavage site of the type III restriction enzyme and the 5' end of the tag is defined by the cleavage site of another restriction enzyme that is closest to the 3' end of the cDNA of the expressed gene.

Preferably, the type III enzyme is *Eco*P15I and the other restriction enzyme is an enzyme capable of cleaving cDNA into fragments with an average length of 200 bp to 300 bp each, as described above. The most preferable enzyme is *Nla*III.

The examples of the tag of the invention are shown in Table 1 below. These tags are longer than conventional SAGE tags or LongSAGE tags, and allow more accurate indetification of the corresponding genes.

The present invention further provides a ditag-oligonucleotide comprising two tags, each of which is derived from a different expressed gene. The ditag-oligonucleotide is produced by the method comprising the following steps:
1) synthesizing a cDNA pool from mRNA of expressed genes using a primer comprising oligo-dT and a recognition sequence of the type III restriction enzyme, followed by digestion of the cDNA pool with another restriction enzyme;
2) purifying fragments comprising poly A sequence from the above cDNA pool, and ligating the fragments to either Linker-A or Linker-B, both of which comprise the recognition sequence of the type III restriction enzyme;
3) digesting the above fragments with the type III restriction enzyme, and ligating the resulting fragment comprising Linker-A to the resulting fragment comprising Linker-B after performing a 3'-filling reaction; and
4) digesting the above ligated fragments with the other restriction enzyme of step 1) to cleave off the Linker sequence, and thereby obtain the ditag-oligonucleotide.

As shown in the above steps, a first recognition site of type III restriction enzyme is incorporated into the target cDNA by the RT primer used for the reverse transcription of cDNA from mRNA and a second recognition site of the type III restriction enzyme is incorporated into the target cDNA by the Linker sequence.

The RT primer is defined by the sequence 5'-N₁₈₋₂₅-**CAGCAG-T**₁₅₋₂₅-3', wherein N₁₈₋₂₅ is an arbitrary nucleotide sequence from 18 to 25 not comprising a sequence 5'-CAGCAG-3' and a sequence 5'-CATG-3'. The 5' end ofRT primer may be modified, for example by biotin.

The type III restriction enzyme of the invention provides fragments having an overhanging 5' end, which can be easily blunt-ended using a conventional 3' filling reaction. Namely, in the above step 3), the fragment comprising Linker-A and the fragment comprising Linker-B can be easily blunt-ended and thereby allow a random association of the fragments without any reduction in tag size.

In the preferred embodiment of the invention, the type III enzyme is *Eco*P15I and the other restriction enzyme is an enzyme capable of cleaving cDNA into fragments with an average length of 200 bp to 300 bp each, such as *Nla*III.

In the method of producing the ditag-oligonucleotide of the invention, two linkers (Linker-A and Linker-B) are used. The Linker A and the Linker-B are double-stranded DNA different from each other. One end of the double-stranded linker fragment participating in ligation comprises the recognition sequence of the type III restriction enzyme adjacent to the sequence for the ligation.
For example, when the type III restriction enzyme is *Eco*P15I and the other restriction enzyme is *Nla*III, both the linkers comprise the 5'-CAGCAGCATG-3' sequence at the 3' ends of their first strands. The underlined sequence 5'-CAGCAG-3' constitutes one of the recognition sites of *EcoP*15I, and the 5'-CATG-3' sequence constitutes a 3' overhanging single-stranded region to ligate to the fragment generated by *Nla*III. digestion.

The other end of the double-stranded linker fragment not participating in ligation may be modified by a labeling reagent such as FITC (Fluorescein Isothiocyanate; the 5'-end of the first strand) and by an amino moiety (the 3'-end of the second strand).

Thus, the linkers are made by annealing the following first strand of DNA(1) and second strand of DNA(2);
DNA(1): 5'-N₃₀₋₄₀-CAGCAGCATG-3'
DNA(2): 3'-N₃₀₋₄₀-GTCGTC-5'
wherein, N₃₀₋₄₀ of DNA(1) and N₃₀₋₄₀ of DNA(2) are arbitrary nucleotide sequences from 30 to 40 bases, which are complementary to each other, and wherein the 5' end of DNA(1) may be labeled and the 3' end of DNA(2) may be amino-modified.

The present invention further provides a polynucleotide obtained by the ligation of the above ditag-oligonucleotides. Each ditag-oligonucleotide may be cloned and amplified by PCR. The polynucleoide comprises at least 2 ditag-oligonucleotides, and preferably comprises 2 to 200 ditag-oligonucleotides. The ditag-oligonucleotides of the invention are made by a random association of the two tags, and therefore the polynucleotide is also a random concatenation of the tag.

The present invention further provides a method of gene expression analysis comprising analysis of the nucleotide sequence of the polynucleotide, and quantification of the expression level of an expressed gene based on the number of tags corresponding to the expressed gene included in the polynucleotide.

The method of invention comprises the following steps:
1) synthesizing a cDNA pool from mRNA of expressed genes using an RT primer comprising oligo-dT and a recognition sequence of a type III restriction enzyme, followed by digestion of the cDNA pool with another restriction enzyme;
2) purifying fragments comprising poly A sequence from the above cDNA pool, and ligating the fragments to either linker-A or linker-B, both of which comprise the recognition sequence of the type III restriction enzyme;
3) digesting the above fragments with the type III restriction enzyme, and ligating the resulting fragment comprising linker-A to the resulting fragment comprising linker-B after performing a 3'-filling reaction;
4) digesting the above ligated fragments with the other restriction enzyme of step 1) to cleave off the linker sequence, and thereby obtain a ditag-oligonucleotide comprising two tags of more than 25 nucleotides and capable of identifying the expressed gene;
5) ligating the ditag-oligonucleotides to produce a polynucleotide; and
6) analyzing the nucleotide sequence of the above polynucleotide, and quantifying the expression level of an expressed gene based on the number of tags corresponding to the expressed gene included in the polynucleotide.

In the preferred embodiment of the method, the type III enzyme is *Eco*P15I and the other restriction enzyme is an enzyme capable of cleaving cDNA into fragments with an average length of 200 bp to 300 bp each, such as *Nla*III. Regarding the linkers, the double-stranded DNA as described above may be preferably used.

The present invention further provides a kit for isolating a tag comprising more than 25 nucleotides and capable of identifying an expressed gene, comprising the following elements:
a) An RT primer defined by the sequence 5'-N₁₈₋₂₅ not comprising a sequence 5'-CAGCAG-3' and a sequence 5'-CATG-3', and wherein the 5' end of RT primer may be biotinylated.
b) Linker-A and Linker-B, which are double-stranded DNA different from each other and made by annealing the following first strand of DNA(1) and second strand of DNA(2):
   DNA(1): 5'-N₃₀₋₄₀-CAGCAGCATG-3'
   DNA(2): 3'-N₃₀₋₄₀-GTCGTC-5'
   wherein, N₃₀₋₄₀ of (1) and N₃₀₋₄₀ of (2) are arbitrary nucleotide sequences from 30 to 40, which are complementary to each other, and the 5' end of DNA(1) may be labeled and the 3' end of DNA(2) may be amino-modified.
c) primers capable of hybridizing to the above Linker-A or Linker-B

The kit may also comprise a type III restriction enzyme such as *Eco*P15I enzyme and/or another restriction enzyme such as *Nla*III. The kit may further comprise, in addition to the aforementioned elements, other elements necessary for carrying out the gene expression analysis of the present invention. Examples include a labeling reagent, a buffer, magnetic beads, or the like.

### BRIEF DESCRIPTION OF THE DRAWINGS

Fig. 1 shows a scheme of the conventional SAGE^{™} protocol (Velculescu, et al., Science 270: 484-487,1995).

Fig. 2 shows a schematic procedure for the isolation of 26-bp cDNA SuperSAGE tags using *Eco*P15I.

Fig. 3 summarizes the application of SuperSAGE analysis using 26-bp tags as obtained by *Eco*P15I.

Fig. 4 shows an example of an electrophoresis of products after *Eco*P15I digestion (step 6, Fig. 2), visualized by FITC fluorescence. The structure of each fragment is depicted on the right side of the panel. In this example, mRNA molecules derived from rice leaves were used as template for cDNA synthesis.

Fig. 5 shows an example of an electrophoresis (PAGE) of PCR products from step 9 of Fig. 2. The size of the expected PCR product is ca. 97 bp.

Fig. 6 shows an example of an electrophoresis (PAGE) of fragments resulting from *Nla*III digestion of the PCR products (step 10). The size of the ditag is ca. 52 bp.

Fig. 7 shows an example of an electrophoresis of concatenated fragments of ditags (step 11).

Fig. 8 shows an example of an electrophoresis of colony PCR products (step 14).

Fig. 9 shows an example of the DNA sequence contained in the cloned concatemer (step 15).

Fig. 10 shows the results of RT-PCR using RNAs isolated from *Magnaporthe grisea-*infected rice leaves using 26-bp tag sequences as PCR primer.

Fig. 11 shows the results of RT-PCR in *Nicotiana benthamiana* that were either treated with INF1 elicitor protein from *Phytophthora infestans* or water as a control.

Fig. 12 shows the RT-PCR kinetic study of gene expression of four genes that were identified by SuperSAGE.

### PREFERRED EMBODIMENTS OF THE PRESENT INVENTION

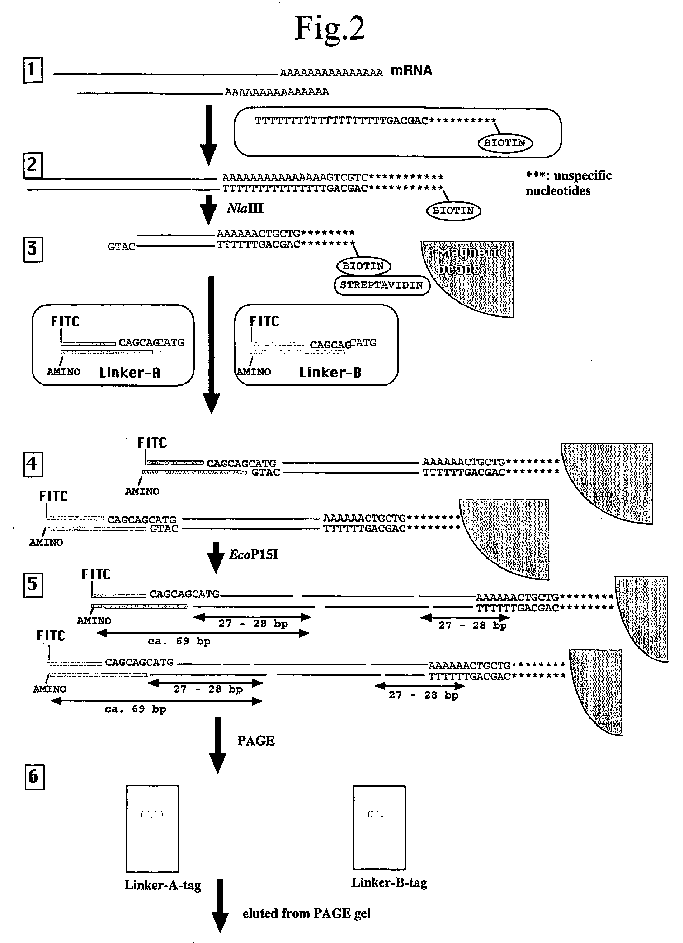

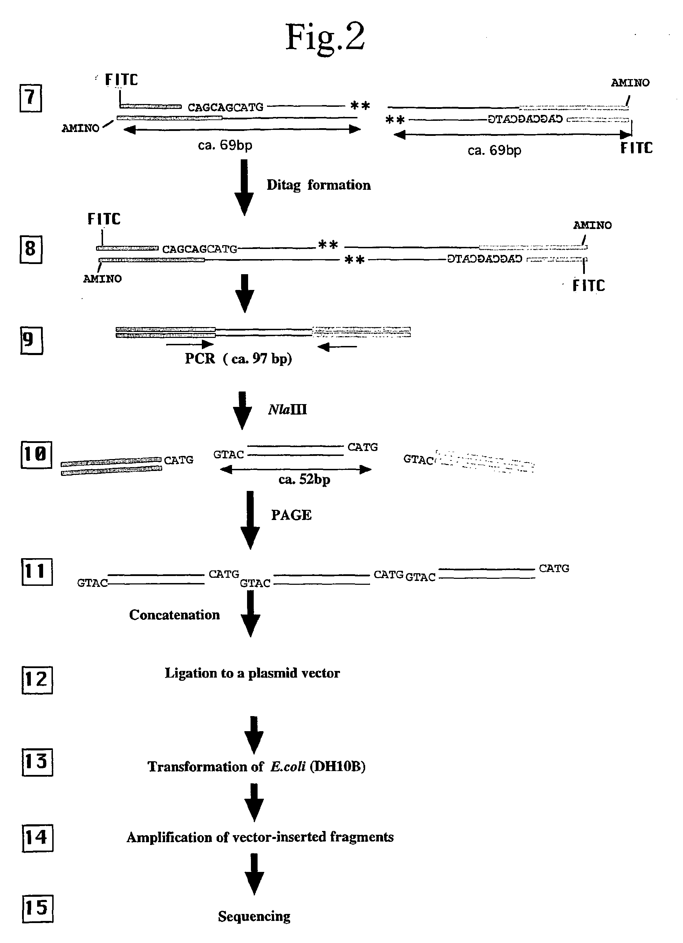

Referring to Fig 2, the preferred embodiment of the present invention using *Eco*P15I as a type III restriction enzyme is described hireinafter.

The type III restriction-modification enzyme *Eco*P15I recognizes two unmethylated inversely oriented 5'-**CAGCAG**-3' sites in the target DNA molecule, and digests 25 to 28 bp apart from the 3'-end of one of the recognition sites. The invention refers to all potential applications of the *Eco*P15I enzyme for the isolation of 25- to 28-bp tag sequences from a defined position of cDNAs.

Double-stranded cDNA is synthesized from mRNA using a biotinylated oligo-dT-anchor primer (hereinafter referred as "RT primer" or reverse transcription primer). This RT primer comprises an arbitrary nucleotide sequence from 18 to 25 bases and the 5'-**CAGCAG**-3' sequence followed by an oligo-dT sequence from 15 to 25 bases. The 5'-**CAGCAG**-3' sequence included in the RT primer constitutes one of the recognition sites of the *Eco*P15I (Fig. 2, step 1). For example, the RT primer comprising a 22-nucleotide sequence and the 5'-**CAGCAG**-3' sequence followed by 19-dT sequence is:
5'-CTGATCTAGAGGTACCGGATCC**CAGCAG**TTTTTTTTTTTTTTTTTTT-3' (SEQ ID NO:1).

Synthesized cDNA is digested by restriction endonuclease *Nla*III, which recognizes the sequence motif 5'-CATG-3'. Only the digested fragments comprising RT primer sequences (biotin-labeled) are captured by streptavidin-coated magnetic beads (Fig. 2, steps 2 and 3).

A double-stranded linker fragment (46 bp) is ligated to the ends of the cDNA fragment (comprising a poly A sequence) captured by magnetic beads. One end of this linker fragment participating in ligation comprises the 5'-**CAGCAG**-3' sequence adjacent to the 5'-CATG-3' sequence in the first strand (Fig. 2, step 4). The 5'-**CAGCAG-**3' sequence constitutes one of the recognition sites of *EcoP*15I, and the 5'-CATG-3'sequence constitutes a 3' overhanging single-stranded region to be ligated to the cohesive end of the fragments generated by *Nla*III digestion. The other end of the double-stranded linker fragment not participating in ligation is modified by FITC (Fluorescein Isothiocyanate; the 5'-end of the first strand) and an amino moiety (the 3'-end of the second strand).

In the present invention, two linkers are used. For example, Linker-A is made by annealing the following two oligonucleotides :
FITC-5'-TTTGGATTTGCTGGTGCAGTACAACTAGGCTTAATAC**AGCAG**CATG-3' (SEQ ID NO:2) and
5'-CTGCTGTATTAAGCCTAGTTGTACTGCACCAGCAAATCCAAA-3'-NH₂ (SEQ ID NO:3).
Linker-B is made by annealing the following two oligonucleotides:
FITC5'-TTTCTGCTCGAATTCAAGCTTCTAACGATGTACG**CAGCAG**CATG-3' (SEQ ID NO:4) and
5'-**CTGCTG**CGTACATCGTTAGAAGCTTGAATTCGAGCAGAAA-3'-NH₂ (SEQ ID NO:5).

The cDNA pool is divided into two halves, with one half ligated to the Linker-A and the other half to the Linker-B, resulting in the "Linker-A ligated cDNAs" and the "Linker-B ligated cDNAs".

For both Linker-A ligated cDNAs and Linker-B ligated cDNAs, DNA fragments bound to the beads are digested with *EcoP*15I (Fig. 2, step 5). *Eco*P15I recognizes a pair of inversely oriented motifs of the sequence 5'-CAGCAG-3', and cleaves 25 to 28 bp apart from the 3'-end of one of the recognition sites. After digestion, two fragments are released from the beads. One is the fragment comprising the linker and the 27- or 28-bp tag fragment (with a total size of 69 or 70 bp), and the other one is a fragment of variable size located in the middle of the double-stranded cDNA fragments. The fragments comprising the poly-A sequence remain bound to the magnetic beads, and do not participate in the following procedure.

The 69- or 70- bp fragment comprising the linker and the 27- or 28-bp tag sequence are visualized by FITC fluorescence under UV radiation, and easily isolated from a polyacrylamide gel by gel excision.

*Eco*P15I provides fragments having an overhanging 5' end, which are easily blunt-ended by the conventional 3'-filling reaction, thereby allowing the random association of the fragments. Therefore, the 69- or 70-bp fragments (linker-tag fragments) originating from Linker-A-ligated cDNAs and Linker-B-ligated cDNAs, respectively, are each blunt-ended by 3'-filling reaction and ligated to each other to form ditags by random association of two tags. The 3'-ends of linker fragments are blocked by an amino-modification, so that ligation occurs only between cDNA tag sequences sides that are blunt-ended (Fig. 2, steps 6, 7).

Resulting ditag molecules are amplified by PCR (Fig. 2, step 9). Examples of PCR primers designed from the linker sequences are shown below:
Ditag primer 1E: biotin-5'-CAACTAGGCTTAATACAGCAGCA-3' (SEQ ID NO:6)
Ditag primer 2E: biotin-5'-CTAACGATGTACGCAGCAGCA-3' (SEQ ID NO:7)
The expected size of the PCR product obtained by PCR using the above primers is ca. 97 bp.

The ca. 97 bp PCR product is digested with *Nla*III (Fig. 2, step 10), thereby releasing ca. 52-bp ditag fragments. These fragments are recovered from the gel, and purified.

Ditag fragments are concatenated by a ligation reaction (Fig. 2, step 11). Concatemers are separated by agarose gel electrophoresis. Fragments larger than 500 bp are eluted from gel and recovered.

Size-separated concatemer fragments are ligated to an appropriate plasmid vector that is predigested with *Sph*I and treated with calf intestine phosphatase (FIG. 2; step 12), and the plasmids transformed into *E. coli* (Fig. 2; step 13).

The insert fragments of the plasmids are PCR amplified (Fig. 2; step 14).

The PCR products are directly sequenced (Fig. 2; step 15). A series of ca. 44- bp ditag sequences are flanked by the *Nla*III recognition sequence CATG. This ca. 52 (44+8)- bp sequence information provides two 26- to 28-bp tag sequences isolated from a defined position of each cDNA.

After counting the number of 26-bp tags in a sample, it is possible to obtain the gene expression profiles as described in the original SAGE^{™} protocol. Since the ditags of the invention are formed by the random association of blunt-ended tags, the result faithfully reflects the expression of each gene.

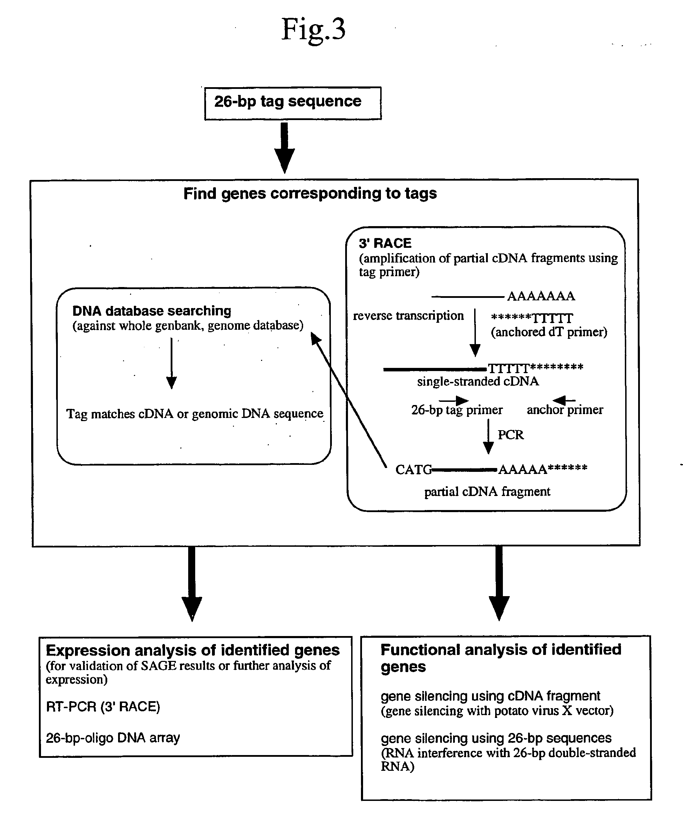

The 26-bp tag sequence contains sufficient information to uniquely identify the gene from which the tag was derived. With the information content in the 26-bp DNA sequence, *in silico* identification of the corresponding gene is facilitated. Even a BLAST search of a 26-bp tag sequence against the entire body of Genbank sequences will show the correct match for the gene from which the tag originated (Fig. 3).

In case the described method is applied for organisms for which no DNA sequence data is available, the 26-bp tag sequence can directly be used as the PCR primer for 3'-RACE to recover the 3'-region of the cDNA. Such cDNA sequence can be used for a BLAST search to identify the gene (Fig. 3).

The 3'-RACE with a 26-bp tag sequence can be directly performed as RT-PCR to quantify the amount of messages for the verification of the gene expression difference between the samples as revealed by the SuperSAGE (Fig. 3).

The 26-bp tag sequence is longer than the minimum size (21 bp) of DNA sequence necessary for triggering "RNA interference" (RNAi) (Elbashir et al. Nature 411: 494-498, 2001). Therefore, double-stranded RNA comprising the tag sequence could be immediately used for the functional analysis to knock out the gene corresponding to the tag. This means that gene expression analysis as performed by SuperSAGE could be directly connected to gene function analysis with the 26-bp tag isolation.

For application in plant species, the 3'-RACE fragment as described above could be cloned into a plant virus vector, and used for "virus-induced gene silencing (VIGS)" (Baulcombe, Curr. Opin. Plant Biol. 2: 109-113,1999), and thus the described SuperSAGE method connects the gene expression analysis to gene function analysis in plants as well.

### EXAMPLES:

The present invention is described in greater detail with reference to the following examples, although the scope of the present invention is not limited to these examples.

### [EXAMPLE 1]

As a proof of principle, 26- and 27-bp tag sequences were isolated from leaves of a lesion-mimic mutant IB2020 of rice (*Oryza sativa* cv. Kakehashi) by the method described above.

### 1) Preparation of mRNA

Total RNA was isolated from leaf blades of rice by a conventional RNA isolation method. From this RNA, 5 µg of mRNA were isolated using an "mRNA Purification Kit" (Amersham Pharmacia). The mRNA was dissolved in 29 µl of DEPC water, and used as source material.

### 2) cDNA synthesis using oligo-dT primer

This mRNA was reverse-transcribed using a "cDNA Synthesis System" (Invitrogen) to generate single-stranded cDNA using the following reverse transcription-primer comprising the 5'-CAGCAG-3' motif that is a recognition sequence of the enzyme *Eco*P15I. Reverse transcription-primer:
5'-CTGATCTAGAGGTACCGGATCCC**AGCAG**TTTTTTTTTTTTTTTTTTT-3'(SEQ ID NO:1)
The product was converted to double-stranded cDNA using the same kit, ethanol precipitated, and dissolved in 20 µL ofLoTE buffer (3mM Tris-HCl pH 7.5, 0.2 mM EDTA).

### 3) NlaIII Digestion

Resulting double-stranded cDNA (20 µL) was digested in 200 µL reaction solution comprising 50 units of *Nla*III (New England BioLabs; NEB) in 1 x NEB Buffer 4 (NEB) containing 0.1 mg/ml BSA at 37 C° for 90 min. After digestion, cDNA was extracted with TE-equilibrated Phenol/ Chloroform/Isoamylalcohol (25-24:1; pH 8.0), ethanol precipitated, and dissolved in 20 µl LoTE buffer.

In each of two Eppendorf tubes, 1 ml streptavidin magnetic beads suspension (Streptavidin Magnesphere Paramagnetic particle, Promega) was transferred. Magnetic beads were washed once with 200 µl 1 x B&W solution (5 mM Tris-HCl pH 7.5, 0.5 mM EDTA, 1M NaCl) using a magnetic separation stand (Magnesphere Technology Magnetic Separation Stand). After washing, 100 µl 1 x B&W buffer and 10 µl cDNA obtained from the above step and 90 µl water were added to each tube, mixed, and incubated at room temperature for 30 min. The cDNA bound by magnetic beads was washed three times with 1 x B&W buffer and three times with LoTE buffer.

### 4) Linker ligation

The following oligonucleotides were synthesized commercially (Qiagen). 
Linker-A1: FITC-5'-TTTGGATTTGCTGGTGCAGTACAACTAGGCTTAATACAGCAGCAT
G-3' (SEQ ID NO:2)
Linker-A2: 5'-**CTGCTG**TATTAAGCCTAGTTGTACTGCACCAGCAAATCCAAA-3'-NH₂ (SEQ ID NO:3)
Linker-B1: FITC5'-TTTCTGCTCGAATTCAAGCTTCTAACGATGTACG**CAGCAG**CATG-3' (SEQ ID NO:4)
Linker-B2:5'-**CTGCTG**CGTACATCGTTAGAAGCTTGAATTCGAGCAGAAA-3'-NH₂ (SEQ ID NO:5)
The 5'-termini of Linker-A2 and Linker-B2 were phosphorylated by T4 polynucleotide kinase (NEB). Linker-A was prepared by annealing Linker-A1 and phosphorylated Linker-A2, and Linker-B by annealing Linker-B1 and phosphorylated Linker-B2. Both Linker-A and Linker-B harbor the *Eco*P15I recognition sequence (5'-**CAGCAG**-3').

To each of the two tubes containing cDNA bound to magnetic beads, 17 µl LoTE, 3 µl 5 X ligase buffer (Invitrogen) and 1 µg either of Linker-A or Linker-B were added. After mixing, tubes were incubated at 50°C for 2 min and left at room temperature for 15 min. Subsequently, 2 µl T4 DNA ligase (5 units/µl, Invitrogen) was added to the tube and incubated at 16°C for 90 min. After linker ligation, the beads were washed 4 times with 1 X B&W, 3 times with LoTE buffer and once with sterile distilled water.

### 5) EcoP15I digestion

Linker-ligated cDNA on the magnetic beads was digested with 10 units *Eco*P15I in 100 µl reaction mixture (10 mM Tris-HCl pH 8.0,10 mM KCl,10 mM MgCl₂, 0.1 mM EDTA, 0.1 mM DTT, 5 µg/ml BSA, 2 mM ATP). Tubes were incubated at 37°C for 90 min.

### 6) Purification of linker-tag fragments

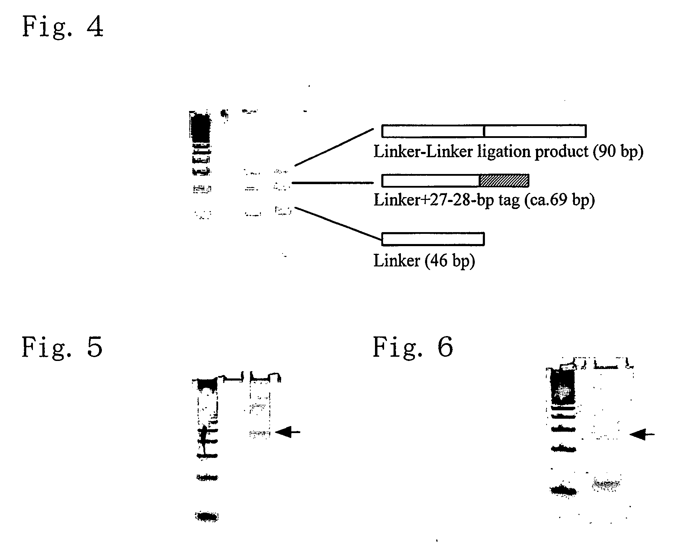

After *Eco*P15I digestion, the tubes were placed on a magnetic stand. After removal of the biotinylated outermost 3'-terminal fragment by streptavidin coated magnetic beads, the unbound fraction containing the linker-tag fragments was collected, and transferred to a new tube. Collected solution was extracted by Phenol/Chloroform/ Isoamylalcohol (25:24:1; pH 8.0), ethanol precipitated, dissolved in 10 µl LoTE buffer, and separated by 8% polyacrylamide gel electrophoresis. In adjacent lanes, size marker (20 bp ladder, TAKARA) prestained with SYBR green (FMC) was loaded. After electrophoresis, the gel was placed on an UV illuminator, and the linker-tag fragment of ca. 69 bp in size was visualized by its FITC-mediated fluorescence (Fig 4). Two additional fragments presumably having originated from linker-linker ligate (ca. 90 bp) and single linker fragments (46 bp) were also visualized. In case the FITC-fluorescence gave too weak signal, the gel could be stained with SYBR green (FMC) to visualize the linker-tag fragment. The ca. 69-bp linker-tag fragment was cut out from the gel and placed into a 0.5 ml tube with a pinhole in the bottom made by a syringe needle. The 0.5 ml tube was placed inside a 2 ml tube, and centrifuged at 15000 rpm for 2 min. To the small gel pieces collected at the bottom of the 2 ml tube, LoTE buffer (300 µl) was added, and they were incubated at 37°C for 2 hrs, followed by incubation at 65 °C for 15 min. The gel suspension was transferred to a SpinX column (Coaster) and centrifuged at 15000 rpm for 2 min. Recovered solution was extracted once by Phenol/Chloroform/Isoamylalcohol (25:24:1; pH 8.0), ethanol precipitated and dissolved in 8 µl LoTE buffer.

### 7) Blunting of tag fragment

The 5'-protrusion of the linker-tag fragments was filled-in by DNA polymerase using a kit (Blunting High; TOYOBO). To the solution containing the linker-tag fragment (8 µl), 1 µl reaction buffer (KOD buffer; TOYOBO) and 1 µl KOD polymerase (TOYOBO) were added, and it was incubated at 72 °C for 2 min.

### 8) Ligation for ditag formation

Linker-tag fragments that had originated from Linker-A and Linker-B were ligated to form the ditag fragments. Equal volumes (2 µl) of blunt-ended Linker-A-tag and Linker-B-tag solutions were mixed, and 6 µl LoTE and 10 µl ligation mixture (Ligation High, TOYOBO) were added. Ligation solution was incubated at 16°C from 4 hrs to overnight.

### 9) Ditag PCR

Resulting ditag solution was diluted five- and ten-fold, and used as template for ditag PCR. The PCR reaction was made in 288 (= 96 x 3) 0.2 ml tubes each containing 50 µL solution containing 1 µL diluted ditag template, 3 units Taq polymerase (AmpliTaq Gold; Applied Biosystems), 1 x AmpliTaq Gold buffer and the PCR primers. The 5'-ends of the PCR primers were biotinylated as follows (commercially synthesized by Qiagen).
Ditag primer 1E: biotin-5'-CAACTAGGCTTAATACAGCAGCA-3' (SEQ ID NO:6)
Ditag primer 2E: biotin-5'-CTAACGATGTACGCAGCAGCA-3' (SEQ ID NO:7)
PCR consisted of initial denaturation at 95°C for 12 min followed by 27-29 cycles of 94 °C for 40 sec and 60°C for 40 sec. Expected size of the amplified ditag fragments was ca. 97 bp (Fig. 5).

### 10) Purification of ditag PCR products

Ditag PCR product (about 300 tubes) was bulked in 10 ml plastic tubes. After Phenol/Chloroform/Isoamylalcohol (25:24:1; pH 8.0) extraction and ethanol precipitation, it was dissolved in 100 µl LoTE buffer. This PCR product was run on a 1.5% low-melting Agarose (SeaPlaque, FMC) gel, and the ca. 97 bp fragment was cut out from gel, which was purified by a Qiagen Gel extraction kit (Qiagen).

### 11) Linker removal by digestion with NlaIII

The purified ca. 97 bp fragment eluted in 121 µl LoTE buffer was digested with 120 units *Nla*III (NEB) in 1 x NEB buffer 4 containing 0.1mg/ml BSA (NEB). After confirmation of digestion by gel electrophoresis (three fragments of 52 bp, 22 bp and 23 bp in size were visualized, Fig. 6), digestion solution was treated by streptavidin magnetic beads at room temperature for 30 min for the removal of linker fragments. After the magnetic beads in the tube were collected by magnetic stand, the supernatant was transferred to a new tube, and the DNA extracted with Phenol/Chloroform/Isoamylalcohol (25:24:1; pH 8.0), ethanol precipitated and dissolved in 10 µl LoTE buffer.

### 12) Purification of 52bp ditag fragment

Recovered DNA was electrophoresed in 12 % polyacrylamide gel. To visualize the fragment, the gel was stained with SYBR green (FMC). A fragment of ca. 52 bp in size was cut out from the gel on a UV transilluminator. DNA was eluted from the gel as describe above. DNA solution eluted from the gel was treated by streptavidin magnetic beads at room temperature for 30 min. Supernatant was collected, and extracted by Phenol/Chloroform/ Isoamylalcohol (25:24:1; pH 8.0), ethanol precipitated and dissolved in 7 µl LoTE buffer.

### 13) Concatemer formation

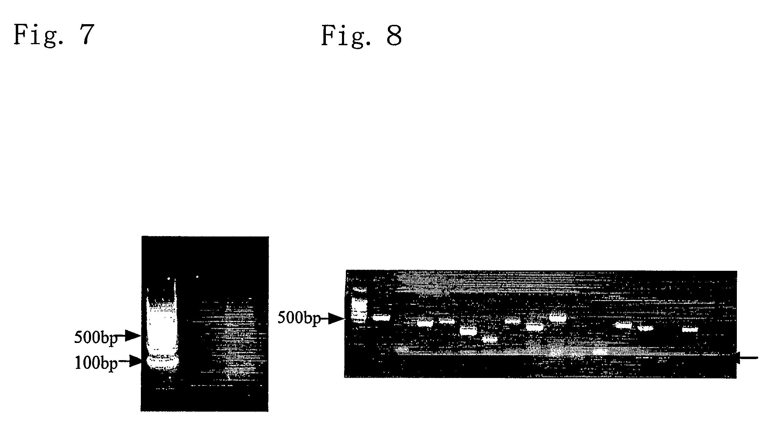

For concatenation of ditag fragments, 7 µl of the ditag solution, 2 µl 5 X ligase buffer, and 5 units T4 ligase (Invitrogen) were mixed and incubated at 16°C for 1-4 hrs. The ligation solution was incubated at 65 °C for 10 min, and loaded on 1 % agarose gel (Fig. 7). DNA fragments of more than 500 bp in size were cut out from gel and eluted. Purified concatemers were dissolved in 6 µl LoTE buffer.

### 14) Vector cloning

Five micrograms of cloning vector (pGEM3Z, Promega) was digested with *Sp*hI, and treated with calf intestine alkaline phosphates (CIAP). For cloning the concatemers, this digested pGEM3Z vector and the ditag concatemers were ligated using T4 ligase (Invitorgen). After 4hrs ligation, reaction solution was extracted with Phenol/Chloroform/Isoamylalcohol (25:24:1), and ethanol precipitated. The pellet was washed 4 times with 70% ethnaol to completely remove salt, and dissolved in 10 µl sterile distilled water.

### 15) Transformation

Electrocompetent *E.coli* cells (DH10B, Invitrogen) were used for the cloning ofplasmids containing the ditag concatemers. In a tube, 40 µl competent cell suspension and 1 µl purified ligated DNA were mixed on ice, and transferred into an electroporation cuvette (0.1cm gap; BioRad). Conditions for electroporation were as recommended by the manufacturer of the competent cells (2.5kV, 25µFD, 100Ω). After electroporation, 1 ml SOC medium (Invitrogen) was added, and the solution was incubated at 37°C for 1 hr. The resulting *E.coli* suspension was plated on an LB medium containing 100 µg/ml Ampicilin, 20 µg/ml X-gal and 0.1 mM IPTG. Plates were incubated at 37°C for 14 hrs.

### 16) Colony PCR and sequencing

White colonies developed on the plates were picked up using toothpicks, and resuspended in the PCR mixture containing 1 X PCR buffer, 0.4mM dNTP, 0.05 unit/µl Taq polymerase (TAKARA), 2 pmols/µl M13-20 primer and 2 pmols/µl M13RV primer. Cloned fragments in the plasmid were amplified by PCR and the fragment size checked by agarose gel electrophoresis (Fig. 8). The amplified PCR fragments were purified using a PCR purification kit (Qiagen).

### 17) Sequencing of concatemers

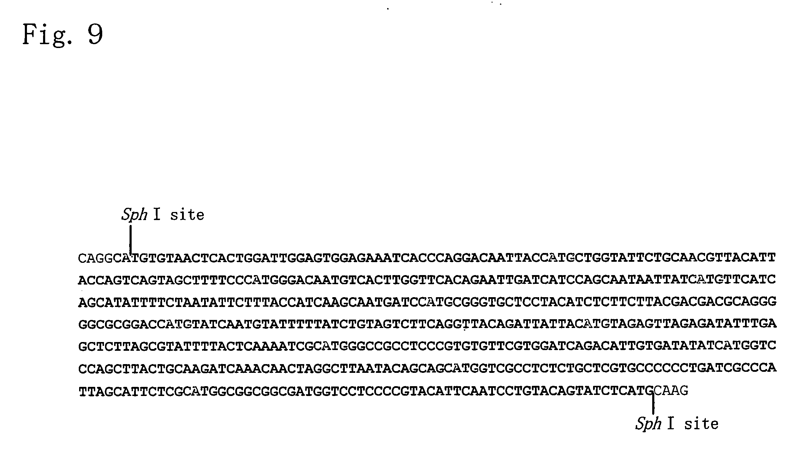

The purified PCR fragments (concatemers) were sequenced with the BigDye terminator (Applied Biosystems) and M13-20 primer. After the sequencing reaction, the DNA was purified by the "Montage SEQ96 Sequencing Reaction Cleanup Kit" (Millipore). DNA sequences were analyzed by the RISA384 capillary DNA sequencer (Shimadzu). An example of the DNA sequences of cloned fragments is shown in Fig. 9. A series of 52- to 54-bp ditags comprising two 26- or 27- bp fragments are delimited by CATG *Nla*III sites.

### 18) Data analysis

From the DNA sequences of concatemers, 26-bp tag sequences were extracted and the number of each tag counted using SAGE^{™}2000 software, supplied by Johns Hopkins University. After sequencing several clones, a gene expression profiling as shown in Table 1 was performed.

**[Table 1] An example of SuperSAGE result obtained from rice leaves. Tag sequences are represented by excluding the shared NlaIII site (CATG).**

| SuperSAGE tag | corresponding SAGE tag | No of tags | Corresponding genes |
|---|---|---|---|
| GATCCGTCTCTCTGGGAGGAAT | GATCCGTCTC | 5 | TC83196 Thiazole biosynthetic enzyme |
| TTGTAATACTCCATCAAAGAGT | TTGTAATACT | 3 | TC82944 catalase |
| CGAAATCGATTCCGAGTTCTCT | CGAAATCGAT | 2 | thioredoxin h |
| ATGGCCTGAGGAAGTGGCTCGC | ATGGCCTGAG | 2 | TC89949 triosephosphate isomerase |
| ATGATGATATACTACACTTGAT | ATGATGATAT | 2 | TC89920 photosystem II 10 kDa polypeptide |
| TTCGGCTTCTTCGTCCAGGCCA | TTCGGCTTCT | 2 | BI809989 chlorophyll a/b-binding protein |
| TTTGTAACTCGTTATATCCTCA | TTTGTAACTC | 2 | TC84234 homeobox gene |
| CTCAAGATGATCGAGGACTACC | CTCAAGATGA | 2 | TC90193 PBZ1 |
| TATATTTATCCATAATATACTC | TATATTTATC | 2 | TC83801 |
| TAATGGTACATATCTCCTTGTT | TAATGGTACA | 2 | TC83132 |
| ACGATTGGTGAATACCCTTGGA | ACGATTGGTG | 1 | AU082990 |
| TTGTGATTCATAACTCTAGTAG | TTGTGATTCA | 1 | N |
| TACATAAATGGAAAAAGAGAGG | TACATAAATG | 1 | genomic DNA AP004997 |
| TTTGACAAGACATTTTCAGTAT | TTTGACAAGA | 1 | TC90371 GF14-d protein |
| GAATCAATACTGTAACTATTAT | GAATCAATAC | 1 | TC83400 thioredoxin M |
| TTCATATGAAATAAAATTATGA | TTCATATGAA | 1 | BI806753 |
| GGGGGCGGCAAGCCGACGATCG | GGGGGCGGCA | 1 | AU070324 |
| TATGTATGTACCTTAATTGTGT | TATGTATGTA | 1 | TC82787 chlorophyll a-b binding protein |
| TGTTACTAGCTGCTACTGTCTA | TGTTACTAGC | 1 | D43299 |
| GCTCTTTCTGTTTGAGGGCCAT | GCTCTTTCTG | 1 | TC83133 |
| GCTGTGTTGTATCTAAACTGTT | GCTGTGTTGT | 1 | TC90306 |
| TAAACAAACCTTCTAAGAGACA | TAAACAAACC | 1 | TC90893 |
| TCGTGGGGTATCTAGGGCTGGA | TCGTGGGGTA | 1 | N |
| AGCATTGTTTTACACGTAATGC | AGCATTGTTT | 1 | genomic DNA AP003249 |
| AAGCTACTTGCTTCCCTTGCTC | AAGCTACTTG | 1 | AA751992 |
| GGTTTCAGCTTGTTCGACTAAT | GGTTTCAGCT | 1 | TC83067 acidic ribosomal protein |
| ATGTAGATCGATGGATGTACCA | ATGTAGATCG | 1 | genomic DNA OSJN00066 |
| CATATGTTGATCAATCTGGAGG | CATATGTTGA | 1 | AP003685 |
| TAACTTACTCTTGGTTAATTAT | TAACTTACTC | 1 | TC83288 |
| TGTAACTCACTGGATTGGAGTG | TGTAACTCAC | 1 | TC88697 |
| GTAATTGTCCTGGGTGATTTCT | GTAATTGTCC | 1 | N |
| CTGGTATTCTGCAACGTTACAT | CTGGTATTCT | 1 | N |
| GGAAAAGCTACTGACTGGTAAT | GGAAAAGCTA | 1 | TC91674 Adenylosuccinate synthetase |
| GGACAATGTCACTTGGTTCACA | GGACAATGTC | 1 | AU071217 |
| ATAATTATTGCTGGATGATCAA | ATAATTATTG | 1 | genomic DNA OSJN00139 |
| TTCATCAGCATATTTTCTAATA | TTCATCAGCA | 1 | TC84963 |
| GATCATTGCTTGATGGTAAAGA | GATCATTGCT | 1 | TC83935 ADP-ribosylation factor |
| CGGGTGCTCCTACATCTCTTCT | CGGGTGCTCC | 1 | AU070302 |
| GTCCGCGCCCCCTGCGTCGTCG | GTCCGCGCCC | 1 | TC83870 |
| TATCAATGTATTTTTATCTGTA | TATCAATGTA | 1 | TC82917 abscisic acid- and stress-inducible protein |
| TAATAATCTGTAACCTGAAGAC | TAATAATCTG | 1 | TC90227 catalase |
| TAGAGTTAGAGATATTTGAGCT | TAGAGTTAGA | 1 | N |
| CGATTTTGAGTAAAATACGCTA | CGATTTTGAG | 1 | TC92829 |
| GGCCGCCTCCCGTGTGTTCGTG | GGCCGCCTCC | 1 | TC90664 1-asparaginase |
| ATATATCACAATGTCTGATCCA | ATATATCACA | 1 | N |
| GTCCCAGCTTACTGCAAGATCA | GTCCCAGCTT | 1 | TC82994 carbonate dehydratase |
| GTCGCCTCTCTGCTCGTGCCCC | GTCGCCTCTC | 1 | TC89722 |
| CGAGAATGCTAATGGGCGATCA | CGAGAATGCT | 1 | genomic DNA AC118672 |
| GCGGCGGCGATGGTCCTCCCCG | GCGGCGGCGA | 1 | TC83113 |
| AGATACTGTACAGGATTGAATG | AGATACTGTA | 1 | TC86544 |
| GCCGCCACCTCGCTGTCGCCGC | GCCGCCACCT | 1 | TC90110 Photosystem I reaction center subunit V |
| CCTAACTTGCTGTAAGCACATT | CCTAACTTGC | 1 | TC90578 |
| GCAAGTTACCAAATGGAAAATA | GCAAGTTACC | 1 | N |
| ATATATATATAAAAAGGATTAG | ATATATATAT | 1 | N |
| CAGGCCGTCGCGCTCGACGTGC | CAGGCCGTCG | 1 | genomic DNA AP005284 |
| AACCAGGATTCACCAGTTCTGG | AACCAGGATT | 1 | TC89467 |
| TATATGTTTTCTAGTTTTCTCT | TATATGTTTT | 1 | genmicDNA AP005056 |
| TAACAAGCAGATTAGAAAGCTG | TAACAAGCAG | 1 | AU089764 |
| CAGCAATACTCTGTAATATTAT | CAGCAATACT | 1 | TC84453 |
| AGTGACTGTTGTTGTAGAGAAC | AGTGACTGTT | 1 | genomic DNA AC090874 |
| GCTTGAGCCCTGGGGCCCTGGC | GCTTGAGCCC | 1 | genomic DNA AP003408 |
| AAGTGTTATACTGTACACTGAT | AAGTGTTATA | 1 | N |
| TGTAACTAAATGAAATGGTGTG | TGTAACTAAA | 1 | TC84198 small GTP-binding protein |
| GAGAAAATTAACGTGAAAAAAA | GAGAAAATTA | I | N |
| TTTGATGCTTGACAGGCAACAT | TTTGATGCTT | 1 | TC84043 remorin |
| GCTATTGCAACTGTGGTGACTG | GCTATTGCAA | 1 | TC84281 |
| TAACAAGCGAGGTGCTTGTGTC | TAACAAGCGA | 1 | TC89910 |
| CCAATACTGGTG | CCAATACTGG | 1 | N |
| GAACTTTGGGATGTAAGCACTG | GAACTTTGGG | 1 | genomicDNA AC103891 |
| TAGCAATGCCAGGGATTTGTAT | TAGCAATGCC | 1 | TC85883 |
| GGATATTGGAATGGGAGTATAA | GGATATTGGA | 1 | genomic DNA AC135258 |
| CTCGACTTCGCCGGCGAGCACG | CTCGACTTCG | 1 | TC86797 sinapyl alcohol dehydrogenase |
| GCACCCACGAGAGAGGGGGCTC | GCACCCACGA | 1 | TC91365 |
| GACGAGGACGCCCCGGCGGGTG | GACGAGGACG | 1 | TC82846 putative HSP70 |
| TGTTAGTATTATGTAACTCTGT | TGTTAGTATT | 1 | TC87007 |
| GCCGCCGAGCCGGAGATACCGG | GCCGCCGAGC | 1 | TC89744 glyceraldehyde-3-phosphate dehydrogenase |
| GGTATGCTTCCATTGTCAGCAG | GGTATGCTTC | 1 | N |
| TAATTTTAATCATCTGTCAGGC | TAATTTTAAT | 1 | TC83170 Photosystem I reaction center subunit II |
| GATATGGACGAGTGTGTTATTT | GATATGGACG | 1 | TC84236 |
| TGTTAAACTTGCCATTCTGATA | TGTTAAACTT | 1 | TC90900 Elongation factor 1-ganuna |
| TTGTCAAGGGCCAGTTGGATGT | TTGTCAAGGG | 1 | AU031603 |
| GCACCTGACACCGACACTGAAG | GCACCTGACA | 1 | AT003457 |
| GGATGCCACTGGATCGAAAAGA | GGATGCCACT | 1 | TC85434 |
| GATAGCAAGTAGTAGCCTGTTT | GATAGCAAGT | 1 | TC84953 |
| CAATCCTTGATCCGAACTGTGC | CAATCCTTGA | 1 | TC85407 |
| ACCCGAGTGTCGGTCTTCAGTT | ACCCGAGTGT | 1 | TC90603 malate dehydrogenase |
| GGAGCTTGACATCAATGGCAGA | GGAGCTTGAC | 1 | TC92018 |
| CAGCGGCGGCTTAGGCGCGTGG | CAGCGGCGGC | 1 | TC84795 |
| GAACCAAAATTTTCAGAGACCC | GAACCAAAAT | 1 | TC84932 |
| CCTCACGGCATCTTAACTAGAA | CCTCACGGCA | 1 | TC83665 |
| ACCATATTCTTGTTGTACTGCC | ACCATATTCT | 1 | TC91249 |
| TTCTTGATAAGGGATCGATTAG | TTCTTGATAA | 1 | genomicDNA AL606615 |
| TACTACTACCTTGTAAACTTTT | TACTACTACC | 1 | BE228969 |
| GTTGAGCAGGAGCTGCCTTCTG | GTTGAGCAGG | 1 | TC90020 S-adenosylmethionine decarboxylase |
| TTCGGCTGCACCGATGCCACCC | TTCGGCTGCA | 1 | TC89652 small subunit of ribulose-1,5-bisphosphate carboxylase |
| GCCGGTGGGCAGGAGCAAGCGG | GCCGGTGGGC | 1 | TC83622 |
| TAGTAGGAAGTTGACATCTTAC | TAGTAGGAAG | 1 | TC91942 heat shock protein 70 |
| AGGGCCGGCGGCAAGCGGCGGG | AGGGCCGGCG | 1 | NP340063 |
| CAGGCAGACAGATCCGTGTGTA | CAGGCAGACA | 1 | TC92072 |

Thus, 26- or 27- bp sequences adjacent to the *Nla*III sites of cDNAs were successfully isolated from rice leaves. The identification tags comprising 26- or 27-bp sequences can be obtained from any cDNA by applying the experimental procedure described herein. This protocol based on the isolation of tags comprising 26- or 27- bp sequences represents a substantial qualitative improvement over the most advanced SAGE^{™} procedure (known as "LongSAGE^{™}") using tags comprising 18- to 21-bp sequences.

In the SuperSAGE method, *Eco*P15I enzyme generates 5'-overhanging ends in the tags that are readily blunt-ended by DNA polymerase. Therefore, ditag formation is made by random association of the blunt-ended tags, thereby ensuring that the number of tags faithfully represents the expression of the genes corresponding to the tags. This property is not available when ditags are formed only between the 21-bp tags comprising compatible 2-bp 3'-overhanging ends in LongSAGE^{™} protocol. To secure the random association of tags in Long SAGE^{™}, the tags should be blunt-ended by removing the 3'-overhanging region. This results in a 2-bp reduction of tag size and thereby reduces the information content of the tags. Thus, the SuperSAGE method described herein is advantageous over the LongSAGE^{™} method from the viewpoint of the larger information content of the tags and the more faithful representation of gene expression profiles.

### [EXAMPLE 2]

To demonstrate that the 26-bp tag size of SuperSAGE allows a highly reliable annotation of the tag to the gene, the following experiment was conducted.

Thirty 26-bp tags were randomly selected from Table 1. These DNA sequences were truncated from the 3'-ends so that the tag sizes became 20, 18 and 15 bp, respectively. The tag size of 15 bp corresponds to that used in the conventional SAGE^{™}. The 18-bp or 20-bp tags were extracted from the tag of LongSAGE^{™}, when the linker-tag fragments were ligated to each other with and without end-blunting, respectively. Using each of the tags of different sizes, a BLAST search was conducted from the entire body of DNA sequences deposited in Genbank representing DNA sequences from more than 130,000 species. The number of species that contained DNA sequences showing a perfect match to a tag of a given size was counted, and the average and maximum numbers of species were obtained across the 30 tag sequences. The search result are summarized in Table 2.

**[Table 2] Summary of BLAST search of 30 rice SAGE tags for the entire body of Genbank data:**

| | Tag size (bp) | | | |
|---|---|---|---|---|
| | 26 | 20 | 18 | 15 |
| Average number of species with DNA sequence perfectly matching the tag | 1.065 | 1.161 | 1.840 | 4.968 |
| Maximum number of species with DNA sequence perfectly matching the tag | 2 | 4 | 7 | 9 |
| Number of tags for which DNA sequences of more than two species showed perfect matches | 2 | 3 | 10 | 30 |

A number of DNA sequences showed a perfect match to a tag sequence, and an increase in tag size reduced the ambiguity of annotation of a tag to a gene.

The conventional SAGE^{™} tag (15 bp) matched DNA sequences of more than 4 species on average, and with a maximum of 9 species. All of the 30 tags were correlated with two or more species (Table 2). The 18-bp tags matches 1.8 species on average, with a maximum of 7 species. Ten tags out of 30 were correlated with two or more species. The 20-bp tags matched 1.16 species on average, with a maximum of 4 species. Only 3 tags out of 30 were correlated with more than two species, indicating a great improvement over the original SAGE^{™} tag length (15 bp). However, note that 20-bp tag could be extracted only when the linker-tags were ligated without blunting the ends, so that the final results of this method do not necessarily represent accurate gene expression. The 26-bp tags of the SuperSAGE method matched 1.06 species on average, with a maximum of only 2 species. As few as 2 tags out of 30 were correlated with the DNA sequences of more than 2 species. These results clearly show that the information content in the 26-bp DNA sequence provides a great improvement in efficiency of gene annotation of the tags.

The 26-bp tags matched DNA sequences of only one species on average, and in most cases matched a single gene of the particular species. Thus, the annotation of the tag sequence can be carried out almost perfectly. Tag annotation in SuperSAGE can be performed against EST sequence database as well as against whole genome sequences.

The high information content of the 26-bp tag in SuperSAGE allows the simultaneous gene expression analysis of two organisms. As an example, we applied the described method to study the gene expression profiles of rice plants infected with blast disease caused by the fungus *Magnaporthe grisea*. After isolating a total of 12,119 tags from blast-infected rice leaves, each tag was annotated by BLAST search for all the genome sequences of rice and *M. grisea*. As expected, the majority of the tags were annotated to rice genes (Table 3), while 74 tags did not match rice sequences but matched blast sequences (Table 4).

**[Table 3] The 10 most abundantly expressed genes in blast-infected rice leaves as revealed by Super SAGE**

| **Tag*** | **Count** | **Accession No.: Genes** |
|---|---|---|
| TTCGGCTTCTTCGTCCAGGCCA | 122 | D00641: chlorophyll a/b binding protein |
| GATCCGTCTCTCTGGGAGGAAT | 116 | AU172529: thiazole biosynthetic enzyme |
| GCGACGCATCGCCTTCAGCTAA | 114 | X13909: chlorophyll a/b binding protein |
| TGGTGGCTTAGCTCTACGTGTA | 111 | AU174449: glycine rich protein |
| TCGGACAAGTGCGGCAACTGCG | 94 | AF001396: metallothionein |
| TTGTAATACTCCATCAAAGAGT | 86 | D29966: catalase |
| AATTGAGTTCGCTTTGGTTATG | 78 | AF010579: glycine rich protein |
| ATGATGATATACTACACTTGAT | 58 | BE230408: photosystem II 10KDa protein |
| GCGTCCACGCTGACCAACGTCG | 57 | BE230423 : unknown protein |
| TATGTATGTACCTTAATTGTGT | 52 | D00642: chlorophyll a/b binding protein |
| **Total Number of tags (different tags)** | **12,119 (7,546)** | |

| | | |
|---|---|---|
| * Tags represented as a 22-bp sequence excluding the *Nla*III site (CATG). | | |

**[Table 4] A partial list of Magnaporthe grisea genes expressed in blast-infected rice leaves as revealed by SuperSAGE**

| Tag sequence* | Count | Putative gene name** |
|---|---|---|
| CGATCACGAGGGGATGATGGTG | 38 | L20685 : hydrophobin |
| TCAGACACAGGCTGTACAAGGC | 2 | nucleoside-diphosphate kinase |
| TCACGTTTAGAAAGGCGACCCG | 2 | 60S ribosomal protein |
| TTGCCCGTATGTACATAAACAA | 1 | BM865406 :NADH-ubiquinone oxidoreductase |
| CAATTGGTGTTTCTTTGGGTTT | 1 | AF056625 : poly-ubiquitin |
| TCGTCTGTGGCTTCAGTTGCTG | 1 | unknown protein |
| ACGAGCTGATGCGCAAGGATGG | 1 | ABC transporter |
| Total number of putative *M. grisea* tags | 74 | |

| | | |
|---|---|---|
| * Tags represented as a 22-bp sequence excluding the *Nla*III site (CATG). ** Putative gene names were deduced from cDNA sequences or genome sequences of *M.grisea* matched with each tag. | | |

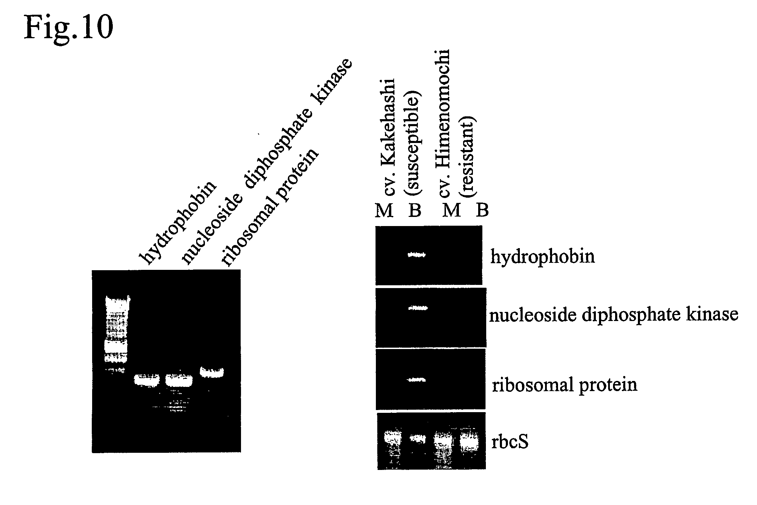

To verify the results obtained by SuperSAGE, cDNA was synthesized by reverse-transcription from the same RNA as above using the following anchored Oligo-dT primer: 5'-GGCCACGCGTCGACTAGTACTTTTTTTTTTTTTTTTT-3' (SEQ ID NO:8). With this cDNA as template, the 26-bp tags for hydrophobin, nucleosid-diphosphate kinase and 60S ribosomal protein genes (Table 4) were used for 3'-RACE together with the following primer: 5'-GGCCACGCGTCGACTAGTAC-3' (SEQ ID NO:9).
Specific PCR products were obtained for all the three genes (Fig. 10, left). The same primer sets were used for RT-PCR in mock-inoculated blast-susceptible cultivar (Kakehashi), blast-inoculated susceptible cultivar (Kakehashi), mock-inoculated blast-resistant cultivar (Himenomochi) and blast-inoculated resistant cultivar (Himenomochi). As expected, PCR products corresponding to the three blast genes were amplified only from susceptible cultivar inoculated with blast fungus (Fig. 10, right), demonstrating the authentity of SuperSAGE annotation.

Quantitative analysis of gene expression of two species in a sample as exemplified here could not be performed by the previous techniques. The use of 26-bp tags for gene expression analysis ("SuperSAGE") will enormously facilitate the gene expression analysis of two or more interacting organisms such as hosts and pathogens simultaneously.

### [EXAMPLE 3]

To demonstrate that SuperSAGE with 26-bp tags can be used for gene expression analysis of organisms for which DNA sequence data are not available, the following experiment was conducted. SuperSAGE was applied to gene expression analysis of a plant species, *Nicotiana benthamiana*, which was treated with either an protein elicitor INF1 from *Phytophthora infestans* (Kamoun et al. Mol. Plant-Microbe Interact. 10:13-20, 1997) or water. The leaves were collected one hour after infiltration of elicitor or water (flooding), and RNA was isolated therefrom.

More than 5000 tags were successfully isolated from each of the samples (Table 5). The ten most abundantly observed tags listed in the Table 5 were used directly for 3'-RACE, and the resulting cDNA were sequenced. BLAST searches of the cDNAs identified the genes corresponding to the tags as given in Table 5.

**[Table 5] The 10 most frequently observed tags in Nicotiana benthamiana leaves revealed by SuperSAGE**

| Tag Sequence* | flooding | INF1 | Corresponding genes** |
|---|---|---|---|
| TGGAAGCTACCTATGTTCGGAT | 130 | 73 | ribulose bisphosphate carboxylase |
| TGGAAGTTGCCTATGTTCGGAT | 51 | 42 | ribulose bisphosphate carboxylase |
| TTCGGGTGCACTGATGCCACTC | 32 | 33 | ribulose bisphosphate carboxylase |
| CAGCAAAGACCAAGAACAGCCC | 16 | 22 | oxygen-evolving protein |
| TTCTCTATGTTCGGATTCTTTG | 22 | 12 | chlorophyll a/b binding protein |
| GTTATGGTGGCAGCCACCCTAG | 19 | 14 | elicitor responsive protein |
| GCATCTTTGGCAACCCTTGCTG | 13 | 11 | photosystemII protein (Psa H) |
| TTTTCTATGTTCGGATTCTTTG | 17 | 8 | chlorophyll a/b binding protein |
| TTTAATCTCTAGTTACATACTG | 15 | 7 | photosystem I subunit PSI-E |
| TATCTTTTCAAGAATACTTTGT | 13 | 5 | photosystemII 10kDa protein |
| **Total number of tags** | **5,095** | **5,089** | |

| | | | |
|---|---|---|---|
| * Tags represented as 22-bp sequences excluding the *Nla*III site (CATG). ** Corresponding genes were deduced from the sequences of amplified cDNA fragments by 3'RACE using 26-bp tag sequence primer. | | | |

By comparing the frequency of each tag in INF1- and flooding-treated samples, tags were identified that were statistically significantly differentially represented in the two samples (Table 6). These tags were directly used for the 3'-RACE, and cDNA recovered was used for BLAST searches. This allowed annotation of most of the tags.

**[Table 6] Differentially expressed genes in INF1- and flooding-treated leaves of Nicotiana benthamiana**

| Tag sequence | Number of tags | | Corresponding genes |
|---|---|---|---|
| | flooding | INF1 | |
| TTTTCTATGTTCGGATTCTTTG | 17 | 8 | chlorophyll a/b binding protein |
| AGGAATAGAGGGCAAGGTGCTC | 11 | 6 | phosphoglycerate kinase (chloroplast) |
| GGCTTTTGCCACTAACTTTGTA | 14 | 4 | chlorophyll a/b binding protein |
| GAGCAATATGAAGACCACAGAG | 11 | 3 | alanine aminotransferase |
| GCTCTTGAAGAGGTTGTGAAAG | 11 | 3 | glycolate oxidase |
| GGCAACAATGCTCTAGAGAAAG | 10 | 2 | ATP synthase (chloroplast) |
| CCTAGCTATTGACTACTGAAGT | 10 | 2 | unknown |
| GTTAAGGTTATTGCTTGGTATG | 7 | 2 | Glyceraldehyde 3-phosphate dehydrogenase (chloroplast)) |
| TTTCCTTGACGATCACTCTTGG | 7 | 2 | PhotosystemII 23kDa protein |
| GTGATTCCCGACGTAGCCGAAG | 6 | 1 | PhotosystemII protein |
| TTGCAACTTCTAGTCAATGACT | 16 | 4 | |
| ATGGCCAAGTAATTTCACCATC | 6 | 1 | |
| AACTCATTAGAGACTCGAAGGG | 6 | 0 | |
| CAACACGAGCACGCACCTCTCT | 0 | 7 | unknown |
| TGCGGGATTCGGTGGTGCCGGA | 5 | 7 | Ubiquitin conjugated protein |
| TTCGGGTGCACTGATGCCACTC | 32 | 33 | Ribluose bisphosphate carboxylase |

| | | | |
|---|---|---|---|
| * Tags represented as 22 bp sequences excluding the *Nla*III site (CATG). ** Corresponding genes were deduced from the sequences of amplified cDNA fragments by 3'RACE using 26-bp tag sequence primer. | | | |

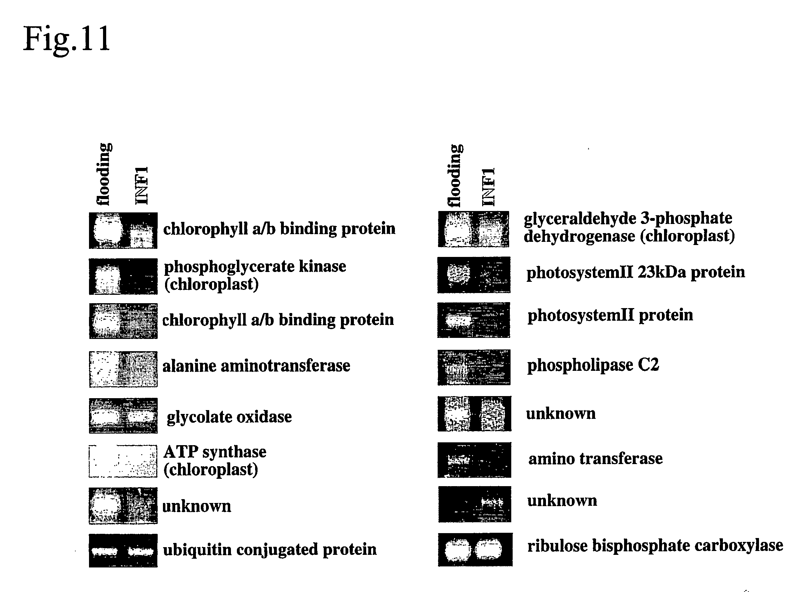

The-26-bp tag sequences were used for RT-PCR together with the anchor primer to verify the results of SuperSAGE shown in Table 6. RT-PCR results (Fig. 11) clearly demonstrate that SuperSAGE results faithfully reflect the gene expression differences between the INF1 and flooding-treated samples.

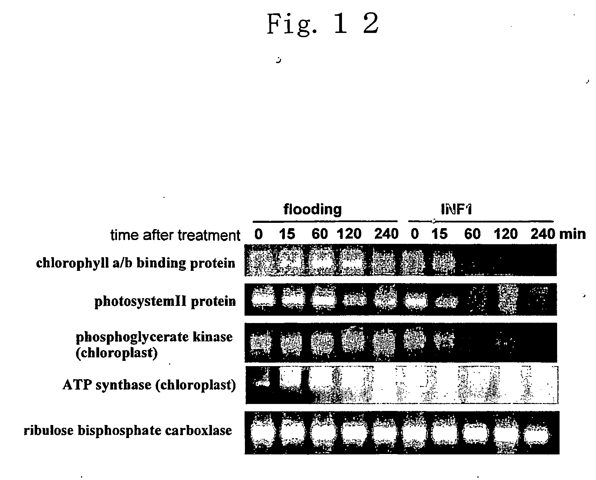

The same 26-bp tag primer can be readily used for RT-PCR for kinetic study of each gene expression (Fig. 12). It was revealed that expression of four tested genes (genes for chlorophyll a/b binding protein, phytosystem II protein, phosphoglycerate kinase, and ATP synthase) were shut off 15 min after the treatment with INF1.

This example demonstrates the great potential of SuperSAGE for gene expression analysis in the organisms for which no DNA sequence data is available. This potential comes from the sufficient size of tags for specific PCR primer and the fact that the gene expression profile obtained by SuperSAGE faithfully represents the real gene expression.

### Free Text of Sequence Listing

SEQ ID NO: 1: RT-primer
SEQ ID NO: 2: First strand of Linker-A
SEQ ID NO: 3: Second strand of Linker-A
SEQ ID NO: 4: First strand of Linker-B
SEQ ID NO: 5: Second strand of Linker-B
SEQ ID NO: 6: Ditag primer (forward)
SEQ ID NO: 7: Ditag primer (reverse)
SEQ ID NO: 8: RT-primer
SEQ ID NO: 9: Primer for 3'-RACE

## Claims

1. Use of a type III restriction enzyme to isolate from cDNA of an expressed gene a tag comprising more than 25 nucleotides and capable of identifying the expressed gene, wherein the 3' end of the tag is defined by a cleavage site of the type III restriction enzyme and the 5' end of the tag is defined by the cleavage site of another restriction enzyme that is closest to the 3' end of the cDNA of the expressed gene.

2. The use of claim 1, wherein the type III restriction enzyme is *Eco*P15I.

3. The use of claim 2, wherein the other restriction enzyme is *Nla*III.

4. A ditag-oligonucleotide comprising two tags each of which is derived from a different expressed gene, wherein
each tag comprises more than 25 nucleotides and is capable of identifying an expressed gene, and the 3' end of the tag is defined by a cleavage site of a type III restriction enzyme and the 5' end of the tag is defined by a cleavage site of another restriction enzyme that is closest to the 3' end of the cDNA of the expressed gene, wherein the two tags are ligated at their 3' ends.

5. The ditag-oligonucleotide of claim 4, produced by a method comprising the following steps:
1) synthesizing a cDNA pool from mRNA of expressed genes using a primer comprising oligo-dT and a recognition sequence of the type III restriction enzyme, followed by digestion of the cDNA pool with another restriction enzyme;
2) purifying fragments comprising poly A sequence from the above cDNA pool, and ligating the fragments to either linker-A or linker-B, both of which comprise the recognition sequence of the type III restriction enzyme;
3) digesting the above fragments with type III restriction enzyme, and ligating the resulting fragment comprising linker-A to the resulting fragment comprising linker-B after performing a 3'-filing reaction; and
4) digesting the above ligated fragments with the other restriction enzyme of step 1) to cleave off the linker sequence, and thereby obtain the ditag-oligonucleotide.

6. The ditag-oligonucleotide of claim 4 or 5, wherein the ditag-oligonucleotide is made by a random association of two tags derived from the different expressed genes.

7. The ditag-oligonucleotide of claim 4, 5 or 6, wherein the type III restriction enzyme is *Eco*P 15I.

8. The ditag-oligonucleotide of claim 7, wherein the other restriction enzyme is *Nla*III.

9. The ditag-oligonucleotide of claim 8, wherein the linker-A and linker-B are doubled-stranded DNA which differ from each other and are made by annealing the following first strand of
DNA(1) and second strand of DNA(2);
DNA(1): 5'-N₃₀₋₄₀-CAGCAGCATG-3'
DNA(2): 3'-N₃₀₋₄₀-GTCGTC-5'
wherein N₃₀₋₄₀ of DNA (1) and N₃₀₋₄₀ of DNA (2) are arbitrary nucleotide sequences from 30 to 40 which are complementary to each other, and wherein the 5' end of DNA(1) may be labeled and the 3' end of DNA(2) may be amino-modified.

10. A polynucleotide comprising at least two ditag-oligonucleotides of any one of claims 4 to 9.

11. The polynucleotide of claim 10, wherein the polynucleotide comprises 2 to 200 ditag-oligonucleotides.

12. A method of gene expression analysis comprising the following steps
1) synthesizing a cDNA pool from mRNA of expressed genes using a primer comprising oligo-dT and a recognition sequence of a type III restriction enzyme, followed by digestion of the cDNA pool with another restriction enzyme;
2) purifying fragments comprising polyA sequence from the above cDNA pool, and ligating the fragments to either linker-A or linker-B, both of which comprise the recognition sequence of the type III restriction enzyme;
3) digesting the above fragments with type III restriction enzyme, and ligating the resulting fragment comprising linker-A to the resulting fragment comprising linker-B after preforming a 3'-filling reaction;
4) digesting the above ligated fragments with the other restriction enzyme of step 1) to cleave off the linker sequence;
5) ligating the ditag-oligonucleotides to produce a polynucleotide; and
6) analysing the nucleotide sequence of the polynucleotide and quantifying the expression level of an expressed gene based on the number of tags corresponding to the expressed gene included in the polynucleotide.

13. A method of gene expression analysis comprising the following steps of:
1) synthesizing a cDNA pool from mRNA of expressed genes using a primer comprising oligo-dT and a recognition sequence of a type III restriction enzyme, followed by digestion of the cDNA pool with another restriction enzyme;
2) purifying fragments comprising poly A sequence from the above cDNA pool, and ligating the fragments to either linker-A or linker-B, both of which comprise the recognition sequence of the type III restriction enzyme;
3) digesting the above fragments with the type III restriction enzyme, and ligating the resulting fragment comprising linker-A to the resulting fragment comprising linker-B after performing a 3'-filling reaction, and
4) digesting the above ligated fragmented with the other restriction enzyme of step 1) to cleave off the linker sequence, and thereby obtain a ditag-oligonucleotide comprising two tags of more than 25 nucleotides and capable of identifying the expressed gene;
5) ligating the ditag-oligonucleotides to produce a polynucleotide;
6) analyzing the nucleotide sequence of the above polynucleotide, and quantifying the expression level of an expressed gene based on the number of tags corresponding to the expressed gene included in the polynucleotide.

14. The method of claim 13, wherein the type III restriction enzyme is *Eco*P15I.

15. The method of claim 14, wherein the other restriction enzyme is *Nla*III.

16. The method of claim 15, wherein the linker-A and linker-B are double-stranded DNA which differ from each other and are made by annealing the following first strand of DNA(1) and second strand of DNA(2);
DNA(1): 5'-N₃₀₋₄₀-CAGCAGCATG-3'
DNA(2): 3'-N₃₀₋₄₀-GTCGTC-5'
wherein, N₃₀₋₄₀ of DNA(1) and N₃₀₋₄₀DNA(2) are arbitrary nucleotide sequences from 30 to 40 which are complementary to each other, and wherein the 5' end of DNA(1) may be labeled and the 3' end of DNA(2) may be amino-modified.

17. A kit for isolating a tag comprising more than 25 nucleotides and capable of identifying an expressed gene, comprising the following elements:
a) an RT primer defined by the sequences 5'-N₁₈₋₂₅-CAGCAG-T₁₅₋₂₅-3', wherein N₁₈₋₂₅ is arbitrary nucleotide sequence from 18 to 25 not comprising a sequence 5'-CAGCAG-3' and a sequence 5'-CATG-3', and wherein the 5' end of RT primer may be biotinylated;
b) linker-A and linker-B which are double-stranded DNA different from each other and made by annealing the following first strand of DNA(1) and second strand of DNA(2):
DNA(1): 5'-N₃₀₋₄₀-CAGCAGCATG-3'
DNA(2): 3'-N₃₀₋₄₀-GTCGTC-5'
wherein N₃₀₋₄₀ of (1) and N₃₀₋₄₀ of (2) are arbitrary nucleotide sequences from 30 to 40 which are complementary to each other, and
the 5' end of DNA(1) may be labeled and the 3' end of DNA(2) may be amino-modified;
c) primers capable of hybridizing to the above linker-A or linker-B.

18. The kit of claim 17, further comprising *Eco*P15I and/or *Nla*III.

## Patentansprüche

1. Verwendung eines TypIII-Restriktionsenzyms, um aus cDNA eines exprimierten Gens einen Tag zu isolieren, welcher mehr als 25 Nukleotide umfasst und in der Lage ist, das exprimierte Gen zu identifizieren, worin das 3'-Ende des Tags durch eine Schnittstelle für das Typ III-Restriktionsenzym definiert ist und das 5'-Ende des Tags durch die dem 3'-Ende der cDNA des exprimierten Gens nächstgelegene Schnittstelle für ein anderes Restriktionsenzym definiert ist.

2. Verwendung nach Anspruch 1, worin das Typ III-Restriktionsenzym *Eco*P15I ist.

3. Verwendung nach Anspruch 2, worin das andere Restriktionsenzym *Nla*III ist.

4. Ditag-Oligonukleotid umfassend zwei jeweils aus einem unterschiedlichen exprimierten Gen abstammende Tags, worin
jeder Tag mehr als 25 Nukleotide umfasst und in der Lage ist, ein exprimiertes Gen zu identifizieren, und das 3'-Ende des Tags durch eine Schnittstelle für ein Typ III-Restriktionsenzym definiert ist und das 5'-Ende des Tags durch eine dem 3'-Ende der cDNA des exprimierten Gens nächstgelegene Schnittstelle für ein anderes Restriktionsenzym definiert ist, worin die zwei Tags an ihren 3'-Enden ligiert sind.

5. Ditag-Oligonukleotid nach Anspruch 4, hergestellt durch ein Verfahren, welches die folgenden Schritte umfasst:
1) Synthese eines cDNA-Pools aus mRNA exprimierter Gene unter Verwendung eines Primers umfassend Oligo-dT und eine Erkennungssequenz für das Typ III-Restriktionsenzym, gefolgt vom Verdau des cDNA-Pools mit einem anderen Restriktionsenzym;
2) Reinigung der Fragmente umfassend PolyA-Sequenzen aus dem obigen cDNA-Pool und Ligation der Fragmente an entweder Linker-A oder Linker-B, die beide die Erkennungssequenz für das Typ III-Restriktionsenzym umfassen;
3) Verdau der obigen Fragmente mit dem Typ III-Restriktionsenzym und Ligation des resultierenden Fragments umfassend Linker-A an das resultierende Fragment umfassend Linker-B nach Durchführung einer 3'-Auffüllreaktion; und
4) Verdau der obigen ligierten Fragmente mit dem anderen Restriktionsenzym aus Schritt 1), um die Linkersequenz abzuspalten und **dadurch** das Ditag-Oligonukleotid zu erhalten.

6. Ditag-Oligonukleotid nach Anspruch 4 oder 5, worin das Ditag-Oligonukleotid durch eine zufällige Assoziation zweier aus den unterschiedlichen exprimierten Genen abstammenden Tags hergestellt wird.

7. Ditag-Oligonukleotid nach den Ansprüchen 4, 5 oder 6, worin das Typ III-Restriktionsenzym *Eco*P15I ist.

8. Ditag-Oligonukleotid nach Anspruch 7, worin das andere Restriktionsenzym *Nla*III ist.

9. Ditag-Oligonukleotid nach Anspruch 8, worin Linker-A und Linker-B doppelsträngige DNA sind, die sich jeweils voneinander unterscheiden und die hergestellt werden durch Zusammenlagern des folgenden ersten DNA(1)-Stranges und des zweiten DNA(2)-Stranges;
DNA(1): 5'-N₃₀₋₄₀-CAGCAGCATG-3'
DNA(2): 3'-N₃₀₋₄₀-GTCGTC-5'
worin N₃₀₋₄₀ aus DNA(1) and N₃₀₋₄₀ aus DNA(2) beliebige Sequenzen von 30 bis 40 Nukleotiden sind, die jeweils zueinander komplementär sind, und worin das 5'-Ende der DNA(1) markiert sein kann und das 3'-Ende der DNA(2) Amino-modifiziert sein kann.

10. Polynukleotid umfassend mindestens zwei Ditag-Oligonukleotide aus einem der Ansprüche 4 bis 9.

11. Polynukleotid aus Anspruch 10, worin das Polynukleotid 2 bis 200 Ditag-Oligonukleotide umfasst.

12. Verfahren zur Genexpressionsanalyse umfassend die folgenden Schritte:
1) Synthese eines cDNA-Pools aus mRNA exprimierter Gene unter Verwendung eines Primers umfassend Oligo-dT und eine Erkennungssequenz für ein Typ III-Restriktionsenzym, gefolgt vom Verdau des cDNA-Pools mit einem anderen Restriktionsenzym;
2) Reinigung der Fragmente umfassend PolyA-Sequenzen aus dem obigen cDNA-Pool und Ligation der Fragmente an entweder Linker-A oder Linker-B, die beide die Erkennungssequenz für das Typ III-Restriktionsenzym umfassen;
3) Verdau der obigen Fragmente mit dem Typ III-Restriktionsenzym und Ligation des resultierenden Fragments umfassend Linker-A an das resultierende Fragment umfassend Linker-B nach Durchführung einer 3'-Auffüllreaktion;
4) Verdau der obigen ligierten Fragmente mit dem anderen Restriktionsenzym aus Schritt 1), um die Linkersequenz abzuspalten;
5) Ligation der Ditag-Oligonukleotide, um ein Polynukleotid herzustellen; und
6) Analyse der Nukleotidsequenz des Polynukleotids und Quantifizierung des Expressionslevels eines exprimierten Gens basierend auf der Anzahl der in das Polynukleotid eingebrachten dem exprimierten Gen entsprechenden Tags.

13. Verfahren zur Genexpressionsanalyse umfassend die folgenden Schritte:
1) Synthese eines cDNA-Pools aus mRNA exprimierter Gene unter Verwendung eines Primers umfassend Oligo-dT und eine Erkennungssequenz für ein Typ III-Restriktionsenzym, gefolgt vom Verdau des cDNA-Pools mit einem anderen Restriktionsenzym;
2) Reinigung der Fragmente umfassend PolyA-Sequenzen aus dem obigen cDNA-Pool, und Ligation der Fragmente an entweder Linker-A oder Linker-B, die beide die Erkennungssequenz für das Typ III-Restriktionsenzym umfassen;
3) Verdau der obigen Fragmente mit dem Typ III-Restriktionsenzym, und Ligation des resultierenden Fragments umfassend Linker-A an das resultierende Fragment umfassend Linker-B nach Durchführung einer 3'-Auffüllreaktion, und
4) Verdau des obigen ligierten Fragments mit dem anderen Restriktionsenzym aus Schritt 1), um die Linkersequenz abzuspalten, und um **dadurch** ein Ditag-Oligonukleotid zu erhalten, welches zwei Tags mit mehr als 25 Nukleotiden umfasst, die in der Lage sind, das exprimierte Gen zu identifizieren;
5) Ligation der Ditag-Oligonukleotide, um ein Polynukleotid herzustellen;
6) Analyse der Nukleotidsequenzen des obigen Polynukleotids, und Quantifizierung des Expressionslevels eines exprimierten Gens basierend auf der Anzahl der in das Polynukleotid eingebrachten dem exprimierten Gen entsprechenden Tags.

14. Verfahren nach Anspruch 13, worin das Typ III-Restriktionsenzym *Eco*P15I ist.

15. Verfahren nach Anspruch 14, worin das andere Restriktionsenzym *Nla*III ist.

16. Das Verfahren nach Anspruch 15, worin Linker-A und Linker-B doppelsträngige DNA sind, die sich jeweils voneinander unterscheiden und die hergestellt werden durch Zusammenlagern des folgenden ersten DNA(1)-Stranges und des zweiten DNA(2)-Stranges;
DNA(1): 5'-N₃₀₋₄₀-CAGCAGCATG-3'
DNA(2): 3'-N₃₀₋₄₀-GTCGTC-5'
worin N₃₀₋₄₀ aus DNA(1) and N₃₀₋₄₀ aus DNA(2) beliebige Sequenzen von 30 bis 40 Nukleotiden sind, die jeweils zueinander komplementär sind, und worin das 5'-Ende der DNA(1) markiert sein kann und das 3'-Ende der DNA(2) Amino-modifiziert sein kann.

17. Kit zur Isolierung eines Tags, welches mehr als 25 Nukleotide umfasst und in der Lage ist, ein exprimiertes Gen zu identifizieren, umfassend die folgenden Elemente:
a) ein RT-Primer definiert durch die Sequenz 5'-N₁₈₋₂₅-CAGCAG-T₁₅₋₂₅-3', worin N₁₈₋₂₅ eine beliebige Sequenz von 18 bis 25 Nukleotiden ist und nicht eine Sequenz 5'-CAGCAG-3' und eine Sequenz 5'-CATG-3' umfasst, und worin das 5'-Ende des RT-Primers biotinyliert sein kann;
b) Linker-A und Linker-B, die aus jeweils voneinander unterschiedlicher doppelsträngiger DNA bestehen und hergestellt werden durch Zusammenlagern des folgenden ersten DNA(1)-Stranges und des zweiten DNA(2)-Stranges;
DNA(1): 5'-N₃₀₋₄₀-CAGCAGCATG-3'
DNA(2): 3'-N₃₀₋₄₀-GTCGTC-5'
worin N₃₀₋₄₀ aus DNA(1) and N₃₀₋₄₀ DNA(2) beliebige Sequenzen von 30 bis 40 Nukleotiden sind, die jeweils zueinander komplementär sind, und worin das 5'-Ende der DNA(1) markiert sein kann und das 3'-Ende der DNA(2) Amino-modifiziert sein kann;
c) Primer, die in der Lage sind, an den obigen Linker-A oder Linker-B zu hybridisieren.

18. Kit nach Anspruch 17, ferner umfassend *Eco*P15I und/oder *Nla*III.

## Revendications

1. Utilisation d'une enzyme de restriction de type III pour isoler à partir de l'ADNc d'un gène exprimé une étiquette comprenant plus de 25 nucléotides et capable d'identifier le gène exprimé, dans laquelle l'extrémité 3' de l'étiquette est définie par un site de clivage d'une enzyme de restriction de type III et l'extrémité 5' de l'étiquette est définie par le site de clivage d'une autre enzyme de restriction qui se situe plus près de l'extrémité 3' de l'ADNc du gène exprimé.

2. L'utilisation selon la revendication 1, dans laquelle l'enzyme de restriction de type III est *Eco*P15I.

3. L'utilisation selon la revendication 2, dans laquelle l'autre enzyme de restriction est *Nla*III.

4. Un oligonucléotide à double étiquette comprenant deux étiquettes, chacune d'elles étant dérivée d'un gène exprimé différent, dans lequel
chaque étiquette comprend plus de 25 nucléotides et est capable d'identifier un gène exprimé, et l'extrémité 3' de l'étiquette est définie par un site de clivage d'une enzyme de restriction de type III et l'extrémité 5' de l'étiquette est définie par un site de clivage d'une autre enzyme de restriction qui se situe plus près de l'extrémité 3' de l'ADNc du gène exprimé, dans lequel les deux étiquettes sont liées à leur extrémité 3'.

5. L'oligonucléotide à double étiquette selon la revendication 4, généré par une méthode comprenant les étapes suivantes :
1) la synthèse d'un pool d'ADNc à partir d'ARNm de gènes exprimés en utilisant une amorce contenant un oligo-dT et une séquence de reconnaissance de l'enzyme de restriction de type III, suivie de la digestion du pool d'ADNc par une autre enzyme de restriction ;
2) la purification des fragments comprenant la séquence polyA du pool d'ADNc ci-dessus, et la ligation des fragments avec un adaptateur A ou un adaptateur B, les deux comprenant la séquence de reconnaissance de l'enzyme de restriction de type III ;
3) la digestion des fragments ci-dessus avec l'enzyme de restriction de type III, et la ligation du fragment résultant comprenant l'adaptateur A avec le fragment résultant comprenant l'adaptateur B après avoir effectué une réaction de remplissage en 3' ; et
4) la digestion des fragments liés ci-dessus avec l'autre enzyme de restriction de l'étape 1) pour cliver la séquence adaptateur et ainsi obtenir l'oligonucléotide à double étiquette.

6. L'oligonucléotide à double étiquette selon la revendication 4 ou 5, dans lequel l'oligonucléotide à double étiquette est construit par l'association aléatoire de deux étiquettes dérivées des différents gènes exprimés.

7. L'oligonucléotide à double étiquette selon la revendication 4, 5 ou 6, dans lequel l'enzyme de restriction de type III est *Eco*P15I.

8. L'oligonucléotide à double étiquette selon la revendication 7, dans lequel l'autre enzyme de restriction est *Nla*III.

9. L'oligonucléotide à double étiquette selon la revendication 8, dans lequel l'adaptateur A et l'adaptateur B sont des ADN double brin qui diffèrent l'un de l'autre et sont construits en appariant le premier brin d'ADN (1) et le second brin d'ADN (2) suivants ;
ADN (1) : 5'-N₃₀₋₄₀-CAGCAGCATG-3'
ADN (2) : 3'-N₃₀₋₄₀-GTCGTC-5'
dans lequel N₃₀₋₄₀ de l'ADN (1) et N₃₀₋₄₀ de l'ADN (2) sont des séquences nucléotidiques arbitraires de 30 à 40 qui sont complémentaires l'une de l'autre, et dans lequel l'extrémité 5' de l'ADN (1) peut être marquée et l'extrémité 3' de l'ADN (2) peut être modifiée par un amino.

10. Un polynucléotide comprenant au moins deux oligonucléotides à double étiquette de n'importe laquelle des revendications 4 à 9.

11. Le polynucléotide selon la revendication 10, dans lequel le polynucléotide comprend de 2 à 200 oligonucléotides à double étiquette.

12. Une méthode d'analyse de l'expression génique comprenant les étapes suivantes :
1) la synthèse d'un pool d'ADNc à partir d'ARNm de gènes exprimés en utilisant une amorce contenant un oligo-dT et une séquence de reconnaissance d'une enzyme de restriction de type III, suivie de la digestion du pool d'ADNc par une autre enzyme de restriction ;
2) la purification des fragments comprenant la séquence polyA à partir du pool d'ADNc ci-dessus, et la ligation des fragments avec soit un adaptateur A soit un adaptateur B, les deux comprenant la séquence de reconnaissance de l'enzyme de restriction de type III ;
3) la digestion des fragments ci-dessus avec l'enzyme de restriction de type III, et la ligation du fragment résultant comprenant l'adaptateur A avec le fragment résultant comprenant l'adaptateur B après avoir effectué une réaction de remplissage en 3' ;
4) la digestion des fragments liés ci-dessus avec l'autre enzyme de restriction de l'étape 1) pour cliver la séquence adaptateur ;
5) la ligation des oligonucléotides à double étiquette pour générer un polynucléotide ; et
6) l'analyse de la séquence nucléotidique du polynucléotide et la quantification du niveau d'expression d'un gène exprimé basée sur le nombre d'étiquettes correspondant au gène exprimé inclues dans le polynucléotide.

13. Une méthode d'analyse de l'expression génique comprenant les étapes suivantes de :
1) synthèse d'un pool d'ADNc à partir d'ARNm de gènes exprimés en utilisant une amorce contenant un oligo-dT et une séquence de reconnaissance d'une enzyme de restriction de type III, suivie de la digestion du pool d'ADNc par une autre enzyme de restriction ;
2) purification des fragments comprenant la séquence polyA du pool d'ADNc ci-dessus, et ligation des fragments avec soit un adaptateur A soit un adaptateur B, les deux comprenant la séquence de reconnaissance de l'enzyme de restriction de type III ;
3) digestion des fragments ci-dessus avec l'enzyme de restriction de type III, et la ligation du fragment résultant comprenant l'adaptateur A avec le fragment résultant comprenant l'adaptateur B après avoir effectué une réaction de remplissage en 3', et
4) digestion des fragments liés ci-dessus avec l'autre enzyme de restriction de l'étape 1) pour cliver la séquence adaptateur, et ainsi obtenir l'oligonucléotide à double étiquette comprenant deux étiquettes de plus de 25 nucléotides et capable d'identifier le gène exprimé ;
5) ligation des oligonucléotides à double étiquette pour générer un polynucléotide ;
6) analyse de la séquence nucléotidique du polynucléotide ci-dessus, et quantification du niveau d'expression d'un gène exprimé basée sur le nombre d'étiquettes correspondant au gène exprimé inclues dans le polynucléotide.

14. La méthode selon la revendication 13, dans laquelle l'enzyme de restriction de type III est *Eco*P15I.

15. La méthode selon la revendication 14, dans lequel l'autre enzyme de restriction est *Nla*III.

16. La méthode selon la revendication 15, dans laquelle l'adaptateur A et l'adaptateur B sont des ADN double brin qui diffèrent l'un de l'autre et sont construits en appariant le premier brin d'ADN (1) et le second brin d'ADN (2) suivants ;
ADN (1) : 5'-N₃₀₋₄₀-CAGCAGCATG-3'
ADN (2) : 3'-N₃₀₋₄₀-GTCGT-5'
dans laquelle N₃₀₋₄₀ de l'ADN (1) et N₃₀₋₄₀ de l'ADN (2) sont des séquences nucléotidiques arbitraires de 30 à 40 qui sont complémentaires l'une de l'autre, et dans laquelle l'extrémité 5' de l'ADN (1) peut être marquée et l'extrémité 3' de l'ADN (2) peut être modifiée par un amino.

17. Un kit pour isoler une étiquette comprenant plus de 25 nucléotides et capable d'identifier un gène exprimé, comprenant les éléments suivants :
a) une amorce RT définie par les séquences 5'-N₁₈₋₂₅-CAGCAG-T₁₅₋₂₅-3', dans laquelle N₁₈₋₂₅ est une séquence nucléotidique arbitraire de 18 à 25 ne comprenant pas de séquence 5'-CAGCAG-3' et de séquence 5'-CATG-3', et dans laquelle l'extrémité 5' de l'amorce RT peut être biotinylée ;
b) un adaptateur A et un adaptateur B qui sont des ADN double brin différents l'un de l'autre et construits en appariant le premier brin d'ADN (1) et le second brin d'ADN (2) suivants :
ADN (1) : 5'-N₃₀₋₄₀-CAGCAGCATG-3'
ADN (2) : 3'-N₃₀₋₄₀-GTCGTC-5'
dans lesquels N₃₀₋₄₀ de (1) et N₃₀₋₄₀ de (2) sont des séquences nucléotidiques arbitraires de 30 à 40 qui sont complémentaires l'une de l'autre, et l'extrémité 5' de l'ADN (1) peut être marquée et l'extrémité 3' de l'ADN (2) peut être modifiée par un amino ;
c) des amorces capables de s'hybrider à l'adaptateur A ou à l'adaptateur B ci-dessus.

18. Le kit selon la revendication 17, comprenant de plus *Eco*P15I et/ou *Nla*III.
